# EUROPEAN PATENT SPECIFICATION

(11) **EP 3 344 747 B1**
(45) Date of publication and mention of the grant of the patent: **09.11.2022**
(21) Application number: 16766432.5
(22) Date of filing: 02.09.2016
(51) Int. Cl.: C12M 3/00

(54) **INTRACELLULAR DELIVERY OF BIOMOLECULES MEDIATED BY A SURFACE WITH PORES**
DURCH EINER OBERFLÄCHE MIT POREN VERMITTELTE INTRAZELLULÄRE FREISETZUNG VON BIOMOLEKÜLEN
ADMINISTRATION INTRACELLULAIRE DE BIOMOLÉCULES MÉDIÉE PAR UNE SURFACE PRÉSENTANT DES PORES

(30) Priority: 04.09.2015 US 201562214820 P; 03.05.2016 US 201662331363 P
(43) Date of publication of application: 11.07.2018
(73) Proprietor: SQZ Biotechnologies Company, Watertown, MA 02472 (US)
(72) Inventor: GILBERT, Jonathan, B., Watertown, MA 02472 (US); FREW, Kirubel, Watertown, MA 02472 (US); BERNSTEIN, Howard, Watertown, MA 02472 (US); SHAREI, Armon, R., Watertown, MA 02472 (US)
(74) Representative: J A Kemp LLP
(86) International application number: PCT/US2016/050287
(87) International publication number: WO 2017/041050

(56) References cited:
- WO-A2-2012/118799
- WILLIAMS A R ET AL: "Filtroporation: A simple, reliable technique for transfection and macromolecular loading of cells in suspension", BIOTECHNOLOGY AND BIOENGINEERING, vol. 65, no. 3, 5 November 1999 (1999-11-05), pages 341-346, XP002223170, WILEY & SONS, HOBOKEN, NJ, US ISSN: 0006-3592, DOI: 10.1002/(SICI)1097-0290(19991105)65:3<341: :AID-BIT12>3.0.CO;2-I
- ARMON SHAREI ET AL: "Cell Squeezing as a Robust, Microfluidic Intracellular Delivery Platform", JOURNAL OF VISUALIZED EXPERIMENTS, no. 81, 7 November 2013 (2013-11-07), XP055182930, DOI: 10.3791/50980
- A. SHAREI ET AL: "A vector-free microfluidic platform for intracellular delivery", PROCEEDINGS OF THE NATIONAL ACADEMY OF SCIENCES, vol. 110, no. 6, 22 January 2013 (2013-01-22), pages 2082-2087, XP055182922, ISSN: 0027-8424, DOI: 10.1073/pnas.1218705110
- CHARLES A DOWNS ET AL: "Cell culture models using rat primary alveolar type I cells", PULMORNARY PHARMACOLOGY & THERAPEUTICS, vol. 24, no. 5, 14 May 2011 (2011-05-14), pages 577-586, XP028284600, ACADEMIC PRESS, GB ISSN: 1094-5539, DOI: 10.1016/J.PUPT.2011.05.005 [retrieved on 2011-05-23]
- SZETO GREGORY LEE ET AL: "Microfluidic squeezing for intracellular antigen loading in polyclonal B-cells as cellular vaccines", SCIENTIFIC REPORTS, vol. 5, 10276, May 2015 (2015-05), XP002764640, ISSN: 2045-2322, DOI: 10.1038/srep10276
- SHAREI ARMON ET AL: "PLASMA MEMBRANE RECOVERY KINETICS OF A MICROFLUIDIC INTRACELLULAR DELIVERY PLATFORM", INTEGRATIVE BIOLOGY, vol. 6, no. 4, 1 April 2014 (2014-04-01), pages 470-475, XP002764641, ISSN: 1757-9694

## Description

### CROSS-REREFENCE TO RELATED APPLICATIONS

This application claims priority to U.S. Provisional Application No. 62/214,820, filed on September 4, 2015 and U.S. Provisional Application No. 62/331,363, filed on May 3, 2016.

### FIELD OF THE INVENTION

The present disclosure relates generally to methods for delivering a compound into a cell by passing a cell suspension through a surface containing pores.

### BACKGROUND

Intracellular delivery is a central step in the research and development of novel therapeutics. Existing technologies aimed at intracellular delivery of molecules rely on electrical fields, nanoparticles, or pore-forming chemicals. However, these methods suffer from numerous complications, including non-specific molecule delivery, modification or damage to the payload molecules, high cell death, the use of materials which may not be generally regarded as safe (GRAS) materials, low throughput, and/or difficult implementation. In addition, these intracellular delivery methods are not effective at delivering molecules to sensitive cell types, such as primary immune cells and stem cells. Thus, there is an unmet need for intracellular delivery techniques that are highly effective at delivering a range of molecules to a variety of cell types. In addition, techniques that allow for rapid, high throughput intracellular delivery can be applied more effectively to large scale clinical, manufacturing, and drug screening applications. References that describe methods of using channels to deliver compounds to cells include SHAREI et al.: "A vector-free microfluidic platform for intracellular delivery",PROCEEDINGS OF THE NATIONAL ACADEMY OF SCIENCES, vol. 110, no. 6, 2013, pages 2082-2087, SHAREI et al: "Plasma membrane recovery kinetics of a microfluidic intracellular delivery platform", INTEGRATIVE BIOLOGY, vol. 6, no. 4, 2014, pages 470-475, WO2013059343 and WO2015023982.

### BRIEF SUMMARY OF THE INVENTION

Certain aspects of the present disclosure include a method for delivering a compound into a cell, the method comprising passing a cell suspension through a surface containing pores, wherein said pores deform the cell thereby causing a perturbation of the cell such that the compound enters the cell, wherein said cell suspension is contacted with the compound. In some embodiments, the surface is a membrane. In some embodiments, the surface is a filter. In some embodiments, the surface is a microsieve. In some embodiments, the surface is a tortuous path surface. In some embodiments, the surface comprises a material selected from one of polycarbonate, polymer, silicon, glass, metal, cellulose nitrate, cellulose acetate, nylon, polyester, polyethersulfone, polytetrafluorethylene, and ceramic. In some embodiments, the entry to the pore is wider than the pore, narrower than the pore, or the same width as the pore. In some embodiments, the surface is manufactured using a method selected from one of etching, track-etching, lithography, laser ablation, stamping, micro-hole punching, polymeric-sponge, direct foaming, extrusion, and hot embossing.

In some embodiments that can be combined with the previous embodiments, the pores size is a function of the cell diameter. In some embodiments, the pore size is about 20%, about 30%, about 40%, about 50%, about 60%, about 70%, about 80%, about 90%, or about 99% of the cell diameter. In some embodiments, the surface cross-sectional width ranges from about 1mm and about 1m. In some embodiments, the pore cross-sectional width ranges from about 0.01µm-about 300µm. In some embodiments, the pore cross-sectional width ranges from about 0.01- about 35µm. In some embodiments, the pore size is about 0.4µm, about 4µm, about 5µm, about 8µm, about 10µm, about 12µm, or about 14µm. In some embodiments, the pore size is about 200µm. In some embodiments, the pores are heterogeneous in size. In some embodiments, the heterogeneous pore sizes vary from 10-20%. In some embodiments, the pores are homogeneous in size.

In some embodiments that can be combined with the previous embodiments, the pores have identical entrance and exit angles. In some embodiments, the pores have different entrance and exit angles. In some embodiments, the pore cross-sectional shape is selected from one of circular, round, square, star, triangle, polygonal, pentagonal, hexagonal, heptagonal, and octagonal. In some embodiments, the cross-sectional shape at the entrance to the pore is different from the cross-sectional shape at the exit from the pore (e.g, circular at the entrance and square at the exit). In some embodiments, the pore cross-sectional shape is selected from cylindrical or conical. In some embodiments, the pore edge is smooth. In some embodiments, the pore edge is sharp. In some embodiments, the pore passage is straight. In some embodiments, the pore passage is curved. In some embodiments, the pores comprise about 10-80% of the total surface area. In some embodiments, the surface comprises about 1.0×10⁵ to about 1.0×10³⁰ total pores. In some embodiments, the surface comprises about 10 to about 1.0×10¹⁵ pores per mm² surface area. In some embodiments, the pores are distributed in parallel. In some embodiments, surfaces with pores are stacked on top of each other. In some embodiments, multiple surfaces are distributed in series. In some embodiments, the pore distribution is ordered. In some embodiments, the pore distribution is random. In some embodiments, the surface thickness is uniform. In some embodiments, the surface thickness is variable. In some embodiments, the surface is about 0.01µm to about 5m thick. In some embodiments, the surface is about 10µm thick. In some embodiments, the surface is <1µm thick.

In some embodiments that can be combined with the previous embodiments, the surface is coated with a material. In some embodiments, the material is Teflon. In some embodiments, the material comprises an adhesive coating that binds to cells. In some embodiments, the material comprises a surfactant. In some embodiments, the material comprises an anticoagulant. In some embodiments, the material comprises a protein. In some embodiments, the material comprises adhesion molecules. In some embodiments, the material comprises antibodies. In some embodiments, the material comprises factors that modulate cellular function. In some embodiments, the material comprises nucleic acids. In some embodiments, the material comprises lipids. In some embodiments, the material comprises carbohydrates. In some embodiments, the material comprises a complex. In some embodiments, the complex is a lipid-carbohydrate complex. In some embodiments, the material comprises transmembrane proteins. In some embodiments, the material is covalently attached to the surface. In some embodiments, the material is non-covalently attached to the surface. In some embodiments, the surface is hydrophilic. In some embodiments, the surface is hydrophobic. In some embodiments, the surface is charged.

In some embodiments that can be combined with the previous embodiments, the cell suspension comprises mammalian cells. In some embodiments, the cell suspension comprises a mixed cell population. In some embodiments, the cell suspension is whole blood. In some embodiments, the cell suspension is lymph. In some embodiments, the cell suspension comprises peripheral blood mononuclear cells. In some embodiments, the cell suspension comprises a purified cell population. In some embodiments, the cell is an immune cell, a cell line cell, a stem cell, a tumor cell, a fibroblast, a skin cell, a neuron, a red blood cell or a platelet. In some embodiments, the immune cell is a lymphocyte, T cell, B cell, dendritic cell, monocyte, macrophage, eosinophil, basophil, NK cell, NKT cell, mast cell or neutrophil. In some embodiments, the cell is a mouse, dog, cat, horse, rat, goat, or rabbit cell. In some embodiments, the cell is a human cell.

In some embodiments that can be combined with the previous embodiments, the compound comprises a nucleic acid. In some embodiments, the compound comprises a nucleic acid encoding DNA, recombinant DNA, cDNA, genomic DNA, RNA, siRNA, mRNA, miRNA, IncRNA, tRNA, shRNA, or self-amplifying mRNA. In some embodiments, the compound is a peptide nucleic acid. In some embodiments, the compound comprises a transposon. In some embodiments, the compound is a plasmid. In some embodiments, the compound comprises a protein-nucleic acid complex. In some embodiments, the compound comprises a Cas9 protein and a guide RNA or donor DNA. In some embodiments, the compound comprises nucleic acid encoding for a Cas9 protein and a guide RNA or donor DNA. In some embodiments, the compound comprises a protein or peptide. In some embodiments, the compound comprises a nuclease, TALEN protein, Zinc finger nuclease, mega nuclease, CRE recombinase, FLP recombinase, R recombinase, integrase, or transposase. In some embodiments, the compound is an antibody. In some embodiments, the compound is a transcription factor. In some embodiments, the compound is a small molecule. In some embodiments, the compound is a nanoparticle. In some embodiments, the compound is a chimeric antigen receptor. In some embodiments, the compound is a fluorescently tagged molecule. In some embodiments, the compound is a liposome. In some embodiments, the compound is a virus.

In some embodiments that can be combined with the previous embodiments, said cell suspension is contacted with the compound before, concurrently, or after passing through the pore. In some embodiments, the compound to be delivered is coated on the surface. In some embodiments, the method is performed between 0°C-45°C. In some embodiments, the cells are passed through the pores by positive pressure or negative pressure. In some embodiments, the cells are passed through the pores by constant pressure or variable pressure. In some embodiments, pressure is applied using a syringe. In some embodiments, pressure is applied using a pump. In some embodiments, pressure is applied using a vacuum. In some embodiments, the cells are passed through the pores by capillary pressure. In some embodiments, the cells are passed through the pores by hydrostatic pressure. In some embodiments, the cells are passed through the pores by blood pressure. In some embodiments, the cells are passed through the pores by g-force. In some embodiments, the cells are passed through the pores under a pressure ranging from about 0.05psi to about 500psi. In some embodiments, the cells are passed through the pores under a pressure of about 2psi. In some embodiments, the cells are passed through the pores under a pressure of about 2.5psi. In some embodiments, the cells are passed through the pores under a pressure of about 3psi. In some embodiments, the cells are passed through the pores under a pressure of about 5psi. In some embodiments, the cells are passed through the pores under a pressure of about 10psi. In some embodiments, the cells are passed through the pores under a pressure of about 20psi. In some embodiments, fluid flow directs the cells through the pores. In some embodiments, fluid flow is turbulent flow prior to the cells passing through the pore. In some embodiments, fluid flow through the pore is laminar flow. In some embodiments, fluid flow is turbulent flow after the cells pass through the pore. In some embodiments, the cells pass through the pores at a uniform cell speed. In some embodiments, the cells pass through the pores at a fluctuating cell speed. In some embodiments, the cells pass through the pores at a speed ranging from about 0.1mm/s to about 20m/s. In some embodiments, the surface is contained within a larger module. In some embodiments, the surface is contained within a syringe.

In some embodiments that can be combined with the previous embodiments, the cell suspension comprises an aqueous solution. In some embodiments, the aqueous solution comprises cell culture medium, PBS, salts, sugars, growth factors, animal derived products, bulking materials, surfactants, lubricants, vitamins, proteins, chelators, and/or an agent that impacts actin polymerization. In some embodiments, the agent that impacts actin polymerization is Latrunculin A, cytochalasin, and/or Colchicine. In some embodiments, the cell culture medium is DMEM, OptiMEM, IMDM, RPMI, or X-VIVO. In some embodiments, the viscosity of the cell suspension ranges from about 8.9×10⁻⁴ Pa.s to about 4.0×10⁻³ Pa.s. In some embodiments, the method further comprises the step of passing the cell through an electric field generated by at least one electrode in proximity to the surface.

Certain aspects of the present disclosure include a device for delivering a compound into a cell, comprising a surface containing pores, wherein said pores are configured such that a cell suspended in a solution can pass through, wherein said pores deform the cell thereby causing a perturbation of the cell such that the compound enters the cell. In some embodiments, the surface is a membrane. In some embodiments, the surface is a filter. In some embodiments, the surface is a tortuous path surface. In some embodiments, the surface comprises a material selected from one of polycarbonate, polymer, silicon, glass, metal, cellulose nitrate, cellulose acetate, nylon, polyester, polyethersulfone, polytetrafluorethylene, and ceramic. In some embodiments, the entry to the pore is wider than the pore, narrower than the pore, or the same width as the pore. In some embodiments, the surface is manufactured using a method selected from one of etching, track-etching, lithography, laser ablation, stamping, micro-hole punching, polymeric-sponge, direct foaming, extrusion, and hot embossing.

In some embodiments that can be combined with the previous embodiments, the pores size is a function of the cell diameter. In some embodiments, the pore size is about 20%, about 30%, about 40%, about 50%, about 60%, about 70%, about 80%, about 90%, or about 99% of the cell diameter. In some embodiments, the surface cross-sectional width ranges from about 1mm and about 1m. In some embodiments, the pore cross-sectional width ranges from about 0.01µm-about 300µm. In some embodiments, the pore cross-sectional width ranges from about 0.01- about 35µm. In some embodiments, the pore size is about 0.4µm, about 4µm, about 5µm, about 8µm, about 10µm, about 12µm, or about 14µm. In some embodiments, the pore size is about 200µm. In some embodiments, the pores are heterogeneous in size. In some embodiments, the heterogeneous pore sizes vary from 10-20%. In some embodiments, the pores are homogeneous in size.

In some embodiments that can be combined with the previous embodiments, the pores have identical entrance and exit angles. In some embodiments, the pores have different entrance and exit angles. In some embodiments, the pore cross-sectional shape is selected from one of circular, round, square, star, triangle, polygonal, pentagonal, hexagonal, heptagonal, and octagonal. In some embodiments, the pore cross-sectional shape is selected from cylindrical or conical. In some embodiments, the pore edge is smooth. In some embodiments, the pore edge is sharp. In some embodiments, the pore passage is straight. In some embodiments, the pore passage is curved. In some embodiments, the pores comprise about 10-80% of the total surface area. In some embodiments, the surface comprises about 1.0×10⁵ to about 1.0×10³⁰ total pores. In some embodiments, the surface comprises about 10 to about 1.0×10¹⁵ pores per mm² surface area. In some embodiments, the pores are distributed in parallel. In some embodiments, surfaces with pores are stacked on top of each other. In some embodiments, multiple surfaces are distributed in series. In some embodiments, the pore distribution is ordered. In some embodiments, the pore distribution is random. In some embodiments, the surface thickness is uniform. In some embodiments, the surface thickness is variable. In some embodiments, the surface is about 0.01µm to about 5m thick. In some embodiments, the surface is about 10µm thick.

In some embodiments that can be combined with the previous embodiments, the surface is coated with a material. In some embodiments, the material is Teflon. In some embodiments, the material comprises an adhesive coating that binds to cells. In some embodiments, the material comprises a surfactant. In some embodiments, the material comprises an anticoagulant. In some embodiments, the material comprises a protein. In some embodiments, the material comprises adhesion molecules. In some embodiments, the material comprises antibodies. In some embodiments, the material comprises factors that modulate cellular function. In some embodiments, the material comprises nucleic acids. In some embodiments, the material comprises lipids. In some embodiments, the material comprises carbohydrates. In some embodiments, the material comprises a complex. In some embodiments, the complex is a lipid-carbohydrate complex. In some embodiments, the material comprises transmembrane proteins. In some embodiments, the material is covalently attached to the surface. In some embodiments, the material is non-covalently attached to the surface. In some embodiments, the surface is hydrophilic. In some embodiments, the surface is hydrophobic. In some embodiments, the surface is charged.

In some embodiments that can be combined with the previous embodiments, the cell suspension comprises mammalian cells. In some embodiments, the cell suspension comprises a mixed cell population. In some embodiments, the cell suspension is whole blood. In some embodiments, the cell suspension is lymph. In some embodiments, the cell suspension comprises peripheral blood mononuclear cells. In some embodiments, the cell suspension comprises a purified cell population. In some embodiments, the cell is an immune cell, a cell line cell, a stem cell, a tumor cell, a fibroblast, a skin cell, a neuron, or a red blood cell. In some embodiments, the immune cell is a lymphocyte, T cell, B cell, dendritic cell, monocyte, macrophage, eosinophil, basophil, NK cell, NKT cell, mast cell or neutrophil. In some embodiments, the cell is a mouse, dog, cat, horse, rat, goat, or rabbit cell. In some embodiments, the cell is a human cell.

In some embodiments that can be combined with the previous embodiments, the compound comprises a nucleic acid. In some embodiments, the compound comprises a nucleic acid encoding DNA, recombinant DNA, cDNA, genomic DNA, RNA, siRNA, mRNA, miRNA, IncRNA, tRNA, shRNA, or self-amplifying mRNA. In some embodiments, the compound is a peptide nucleic acid. In some embodiments, the compound comprises a transposon. In some embodiments, the compound is a plasmid. In some embodiments, the compound comprises a protein-nucleic acid complex. In some embodiments, the compound comprises a Cas9 protein and a guide RNA or donor DNA. In some embodiments, the compound comprises nucleic acid encoding for a Cas9 protein and a guide RNA or donor DNA. In some embodiments, the compound comprises a protein or peptide. In some embodiments, the compound comprises a nuclease, TALEN protein, Zinc finger nuclease, mega nuclease, CRE recombinase, FLP recombinase, R recombinase, integrase, or transposase. In some embodiments, the compound is an antibody. In some embodiments, the compound is a transcription factor. In some embodiments, the compound is a small molecule. In some embodiments, the compound is a nanoparticle. In some embodiments, the compound is a chimeric antigen receptor. In some embodiments, the compound is a fluorescently tagged molecule. In some embodiments, the compound is a liposome. In some embodiments, the compound is a virus.

In some embodiments that can be combined with the previous embodiments, said cell suspension is contacted with the compound before, concurrently, or after passing through the pore. In some embodiments, the compound to be delivered is coated on the surface. In some embodiments, the device is between 0°C-45°C. In some embodiments, the cells are passed through the pores by positive pressure or negative pressure. In some embodiments, the cells are passed through the pores by constant pressure or variable pressure. In some embodiments, pressure is applied using a syringe. In some embodiments, pressure is applied using a pump. In some embodiments, pressure is applied using a vacuum. In some embodiments, the cells are passed through the pores by capillary pressure. In some embodiments, the cells are passed through the pores by blood pressure. In some embodiments, the cells are passed through the pores by g-force. In some embodiments, the cells are passed through the pores under a pressure ranging from about 0.05psi to about 500psi. In some embodiments, the cells are passed through the pores under a pressure of about 2psi. In some embodiments, the cells are passed through the pores under a pressure of about 2.5psi. In some embodiments, the cells are passed through the pores under a pressure of about 3psi. In some embodiments, the cells are passed through the pores under a pressure of about 5psi. In some embodiments, the cells are passed through the pores under a pressure of about 10psi. In some embodiments, the cells are passed through the pores under a pressure of about 20psi. In some embodiments, fluid flow directs the cells through the pores. In some embodiments, fluid flow is turbulent flow prior to the cells passing through the pore. In some embodiments, fluid flow through the pore is laminar flow. In some embodiments, fluid flow is turbulent flow after the cells pass through the pore. In some embodiments, the cells pass through the pores at a uniform cell speed. In some embodiments, the cells pass through the pores at a fluctuating cell speed. In some embodiments, the cells pass through the pores at a speed ranging from about 0.1mm/s to about 20m/s. In some embodiments, the surface is contained within a larger module. In some embodiments, the surface is contained within a syringe.

In some embodiments that can be combined with the previous embodiments, the cell suspension comprises an aqueous solution. In some embodiments, the aqueous solution comprises cell culture medium, PBS, salts, sugars, growth factors, animal derived products, bulking materials, surfactants, lubricants, vitamins, proteins, chelators, and/or an agent that impacts actin polymerization. In some embodiments, the agent that impacts actin polymerization is Latrunculin A, cytochalasin, and/or Colchicine. In some embodiments, the cell culture medium is DMEM, OptiMEM, IMDM, RPMI, or X-VIVO. In some embodiments, the viscosity of the cell suspension ranges from about 8.9×10⁻⁴ Pa.s to about 4.0×10⁻³ Pa.s. In some embodiments, the device comprises multiple surfaces. In some embodiments, the surface is a Transwell. In some embodiments, at least one electrode is in proximity to the surface to generate an electric field.

Certain aspects of the present disclosure include a cell comprising a perturbation, wherein the cell is produced by passing the cell through a surface containing pores, wherein the pores deform the cell thereby causing the perturbation such that a compound is capable of entering the cell. In some embodiments, the surface is a membrane. In some embodiments, the surface is a filter. In some embodiments, the surface is a tortuous path surface. In some embodiments, the surface comprises a material selected from one of polycarbonate, polymer, silicon, glass, metal, cellulose nitrate, cellulose acetate, nylon, polyester, polyethersulfone, polytetrafluorethylene, and ceramic. In some embodiments, the entry to the pore is wider than the pore, narrower than the pore, or the same width as the pore. In some embodiments, the surface is manufactured using a method selected from one of etching, track-etching, lithography, laser ablation, stamping, micro-hole punching, polymeric-sponge, direct foaming, extrusion, and hot embossing.

In some embodiments that can be combined with the previous embodiments, the pores size is a function of the cell diameter. In some embodiments, the pore size is about 20%, about 30%, about 40%, about 50%, about 60%, about 70%, about 80%, about 90%, or about 99% of the cell diameter. In some embodiments, the surface cross-sectional width ranges from about 1mm and about 1m. In some embodiments, the pore cross-sectional width ranges from about 0.01µm-about 300µm. In some embodiments, the pore cross-sectional width ranges from about 0.01- about 35µm. In some embodiments, the pore size is about 0.4µm, about 4µm, about 5µm, about 8µm, about 10µm, about 12µm, or about 14µm. In some embodiments, the pore size is about 200µm. In some embodiments, the pores are heterogeneous in size. In some embodiments, the heterogeneous pore sizes vary from 10-20%. In some embodiments, the pores are homogeneous in size.

In some embodiments that can be combined with the previous embodiments, the pores have identical entrance and exit angles. In some embodiments, the pores have different entrance and exit angles. In some embodiments, the pore cross-sectional shape is selected from one of circular, round, square, star, triangle, polygonal, pentagonal, hexagonal, heptagonal, and octagonal. In some embodiments, the pore cross-sectional shape is selected from cylindrical or conical. In some embodiments, the pore edge is smooth. In some embodiments, the pore edge is sharp. In some embodiments, the pore passage is straight. In some embodiments, the pore passage is curved. In some embodiments, the pores comprise about 10-80% of the total surface area. In some embodiments, the surface comprises about 1.0×10⁵ to about 1.0×10³⁰ total pores. In some embodiments, the surface comprises about 10 to about 1.0×10¹⁵ pores per mm² surface area. In some embodiments, the pores are distributed in parallel. In some embodiments, surfaces with pores are stacked on top of each other. In some embodiments, multiple surfaces are distributed in series. In some embodiments, the pore distribution is ordered. In some embodiments, the pore distribution is random. In some embodiments, the surface thickness is uniform. In some embodiments, the surface thickness is variable. In some embodiments, the surface is about 0.01µm to about 5m thick. In some embodiments, the surface is about 10µm thick.

In some embodiments that can be combined with the previous embodiments, the surface is coated with a material. In some embodiments, the material is Teflon. In some embodiments, the material comprises an adhesive coating that binds to cells. In some embodiments, the material comprises a surfactant. In some embodiments, the material comprises an anticoagulant. In some embodiments, the material comprises a protein. In some embodiments, the material comprises adhesion molecules. In some embodiments, the material comprises antibodies. In some embodiments, the material comprises factors that modulate cellular function. In some embodiments, the material comprises nucleic acids. In some embodiments, the material comprises lipids. In some embodiments, the material comprises carbohydrates. In some embodiments, the material comprises a complex. In some embodiments, the complex is a lipid-carbohydrate complex. In some embodiments, the material comprises transmembrane proteins. In some embodiments, the material is covalently attached to the surface. In some embodiments, the material is non-covalently attached to the surface. In some embodiments, the surface is hydrophilic. In some embodiments, the surface is hydrophobic. In some embodiments, the surface is charged.

In some embodiments that can be combined with the previous embodiments, the cell is a mammalian cell. In some embodiments, the cell is an immune cell, a cell line cell, a stem cell, a tumor cell, a fibroblast, a skin cell, a neuron, or a red blood cell. In some embodiments, the immune cell is a lymphocyte, T cell, B cell, dendritic cell, monocyte, macrophage, eosinophil, basophil, NK cell, NKT cell, mast cell or neutrophil. In some embodiments, the cell is a mouse, dog, cat, horse, rat, goat, or rabbit cell. In some embodiments, the cell is a human cell.

In some embodiments that can be combined with the previous embodiments, the compound comprises a nucleic acid. In some embodiments, the compound comprises a nucleic acid encoding DNA, recombinant DNA, cDNA, genomic DNA, RNA, siRNA, mRNA, miRNA, IncRNA, tRNA, shRNA, or self-amplifying mRNA. In some embodiments, the compound is a peptide nucleic acid. In some embodiments, the compound comprises a transposon. In some embodiments, the compound is a plasmid. In some embodiments, the compound comprises a protein-nucleic acid complex. In some embodiments, the compound comprises a Cas protein and a guide RNA or donor DNA. In some embodiments, the compound comprises nucleic acid encoding for a Cas protein and a guide RNA or donor DNA. In some embodiments, the compound comprises a Cas9 protein and a guide RNA or donor DNA. In some embodiments, the compound comprises nucleic acid encoding for a Cas9 protein and a guide RNA or donor DNA. In some embodiments, the compound comprises a protein or peptide. In some embodiments, the compound comprises a nuclease, TALEN protein, Zinc finger nuclease, mega nuclease, CRE recombinase, FLP recombinase, R recombinase, integrase, or transposase. In some embodiments, the compound is an antibody. In some embodiments, the compound is a transcription factor. In some embodiments, the compound is a small molecule. In some embodiments, the compound is a nanoparticle. In some embodiments, the compound is a chimeric antigen receptor. In some embodiments, the compound is a fluorescently tagged molecule. In some embodiments, the compound is a liposome. In some embodiments, the compound is a virus. In some embodiments, the cell suspension comprises a cell comprising a cell wall. In some embodiments, the cell is a plant, yeast, fungal, algal, or bacterial cell.

In some embodiments that can be combined with the previous embodiments, said cell is contacted with the compound before, concurrently, or after passing through the pore. In some embodiments, the compound to be delivered is coated on the surface. In some embodiments, the cell is passed through the pore at between 0°C-45°C. In some embodiments, the cell is passed through the pore by positive pressure or negative pressure. In some embodiments, the cell is passed through the pore by constant pressure or variable pressure. In some embodiments, pressure is applied using a syringe. In some embodiments, pressure is applied using a pump. In some embodiments, pressure is applied using a vacuum. In some embodiments, the cell is passed through the pore by capillary pressure. In some embodiments, the cell is passed through the pore by blood pressure. In some embodiments, the cell is passed through the pore by g-force. In some embodiments, the cell is passed through the pore under a pressure ranging from about 0.05psi to about 500psi. In some embodiments, the cell is passed through the pore under a pressure of about 2psi. In some embodiments, the cell is passed through the pore under a pressure of about 2.5psi. In some embodiments, the cell is passed through the pore under a pressure of about 3psi. In some embodiments, the cell is passed through the pore under a pressure of about 5psi. In some embodiments, the cell is passed through the pore under a pressure of about 10psi. In some embodiments, the cell is passed through the pore under a pressure of about 20psi. In some embodiments, fluid flow directs the cell through the pore. In some embodiments, fluid flow is turbulent flow prior to the cell passing through the pore. In some embodiments, fluid flow through the pore is laminar flow. In some embodiments, fluid flow is turbulent flow after the cell passes through the pore. In some embodiments, the cell passes through the pore at a speed ranging from about 0.1mm/s to about 20m/s. In some embodiments, the surface is contained within a larger module. In some embodiments, the surface is contained within a syringe.

In some embodiments that can be combined with the previous embodiments, the cell is in a cell suspension comprising an aqueous solution. In some embodiments, the aqueous solution comprises cell culture medium, PBS, salts, sugars, growth factors, animal derived products, bulking materials, surfactants, lubricants, vitamins, proteins, chelators, and/or an agent that impacts actin polymerization. In some embodiments, the agent that impacts actin polymerization is Latrunculin A, cytochalasin, and/or Colchicine. In some embodiments, the cell culture medium is DMEM, OptiMEM, IMDM, RPMI, or X-VIVO. In some embodiments, the cell was further passed through an electric field generated by at least one electrode in proximity to the surface.

### BRIEF DESCRIPTION OF THE FIGURES

The following drawings form part of the present specification and are included to further demonstrate certain aspects of the present disclosure. The disclosure may be better understood by reference to one or more of these drawings in combination with the detailed description of specific embodiments presented herein.

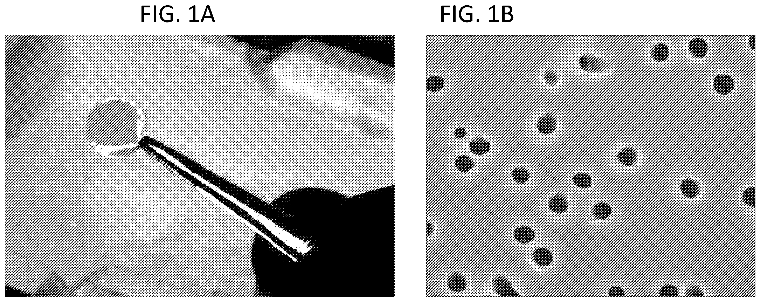

**FIG. 1A** shows a photo of an exemplary polycarbonate filter for use in the examples described. **FIG. 1B** show photos of exemplary polycarbonate filter pores.

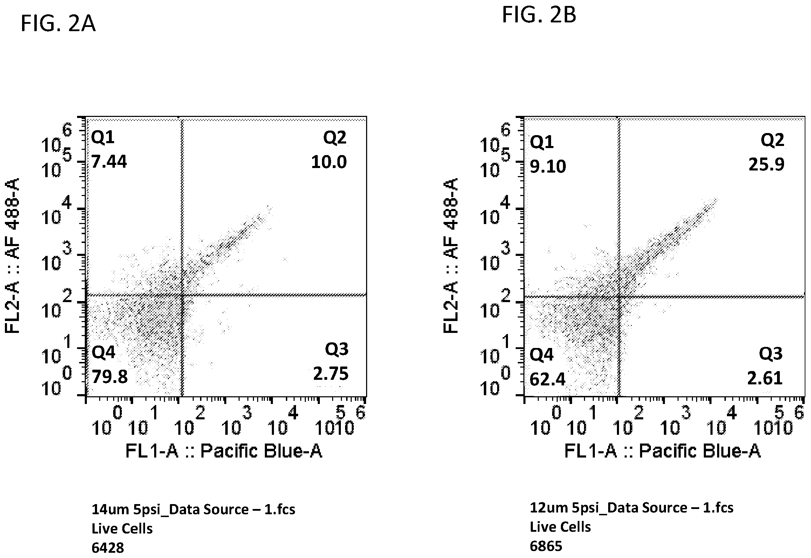

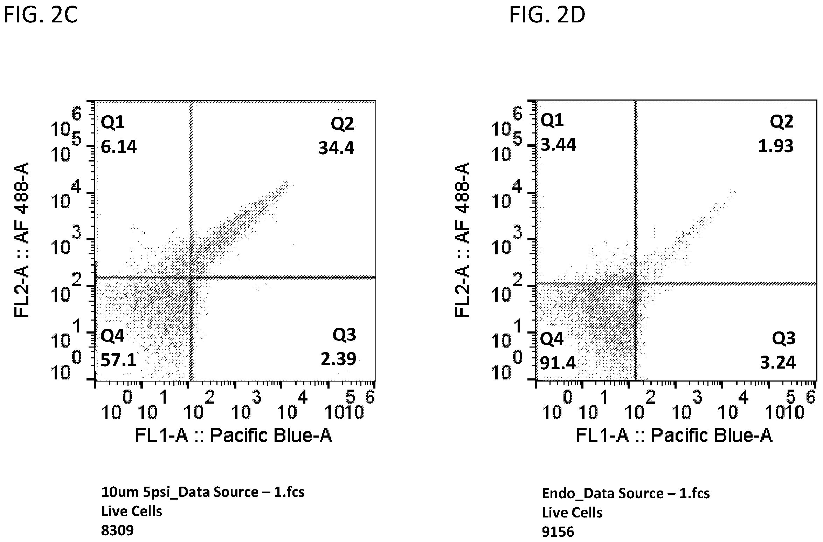

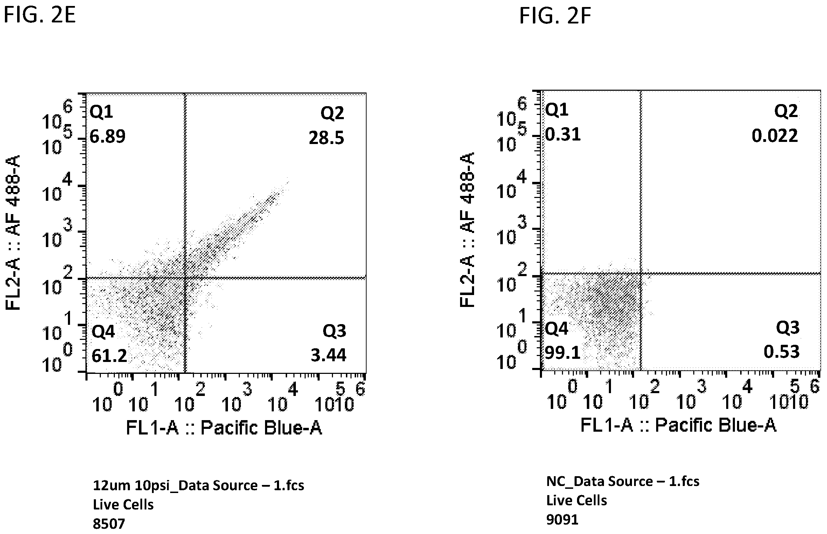

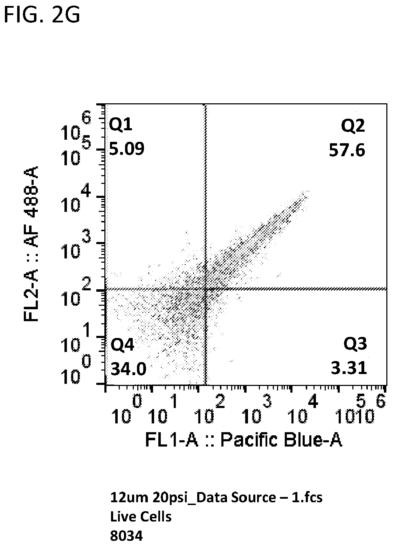

**FIGS. 2A-G** show exemplary FACS plots demonstrating delivery of 3kDa PacBlue and lOkDa Alexa 488 dextran particles to HeLa cells (2×10⁶cells/mL) at low pressure (5psi, 10psi, or 20psi) through 8µm, 10µm, 12µm, or 14µm sized filter pores. **FIGS. 2A-C** depict cells passed through 14µm (**FIG. 2A**), 12µm (**FIG. 2B**), and 10µm (**FIG. 2C**) sized filter pores under a pressure of 5psi. **FIGS. 2E****&****G** depict cells passed through 12µm filter pores under a pressure of 10psi (**FIG. 2E**) and 20psi (**FIG. 2G**). **FIGS. 2D****&****F** depict endocytosis control (**FIG. 2D**) and negative control (**FIG. 2F**) plots.

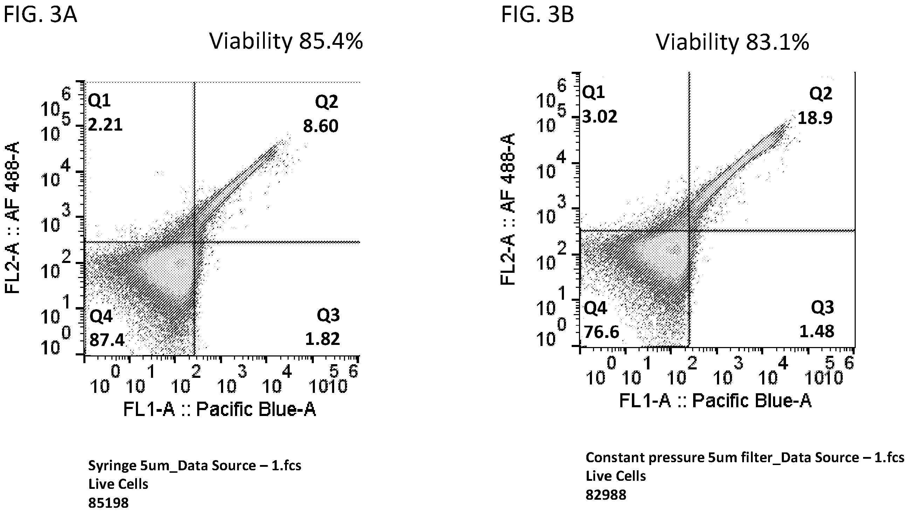

**FIGS. 3A and 3B** show FACS plots demonstrating delivery of 3kDa PacBlue and lOkDa Alexa 488 dextran particles to freshly isolated human T cells (4×10⁶cells/mL), with pressure applied manually via a syringe (**FIG. 3A**) or under a constant pressure of 5psi (**FIG. 3B**) through 5µm sized filter pores. The cell viability frequency is indicated.

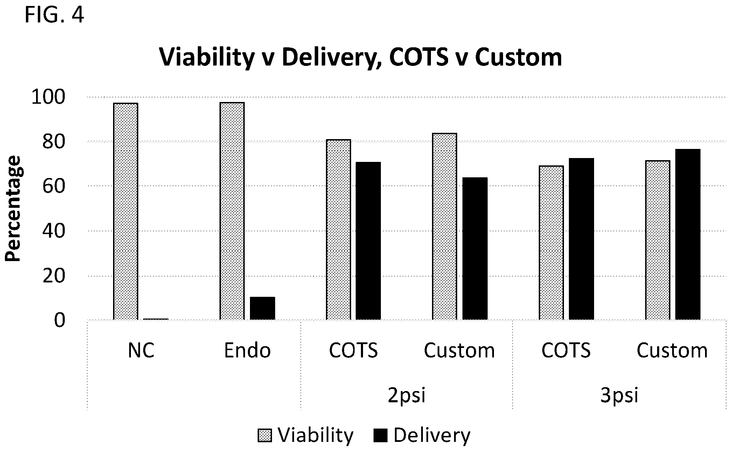

**FIG. 4** shows delivery efficiency and cell viability post-delivery of 3kDa PacBlue dextran particles to HeLa cells mediated by commercial (COTS) filters or custom syringe filters.

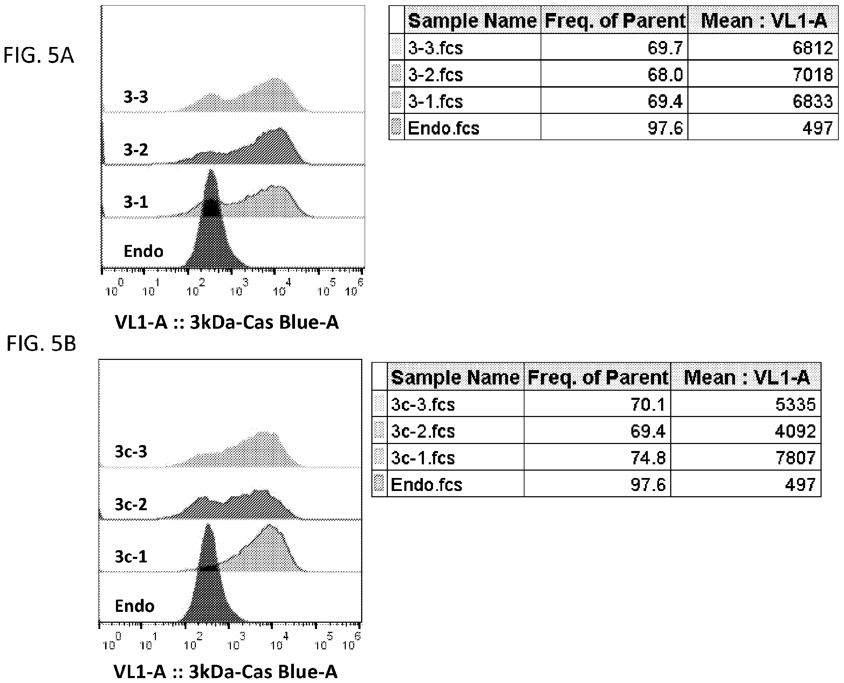

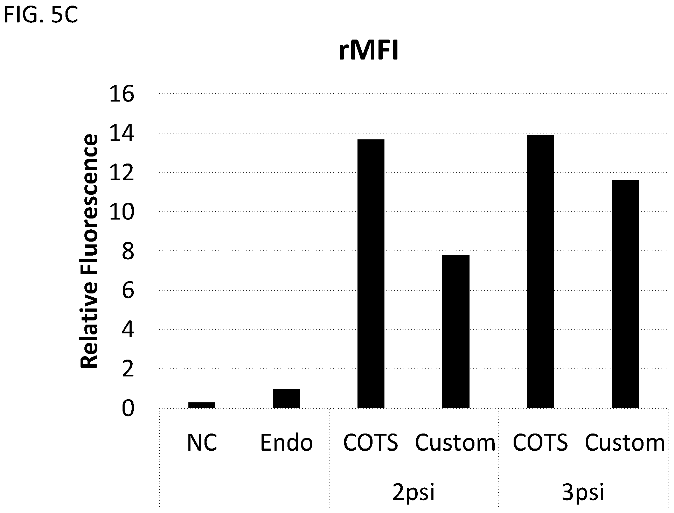

**FIGS. 5A&B** show representative flow cytometry histogram plots demonstrating mean fluorescence intensity (MFI) values for COTS (**FIG. 5A**) or custom syringe filter (**FIG. 5B**) mediated delivery of 3kDa PacBlue dextran particles to HeLa cells at 3psi. **FIG. 5C** shows average relative mean fluorescence intensity (rMFI) values for COTS or custom syringe filter mediated delivery of 3kDa PacBlue dextran particles to HeLa cells at 2psi and 3psi.

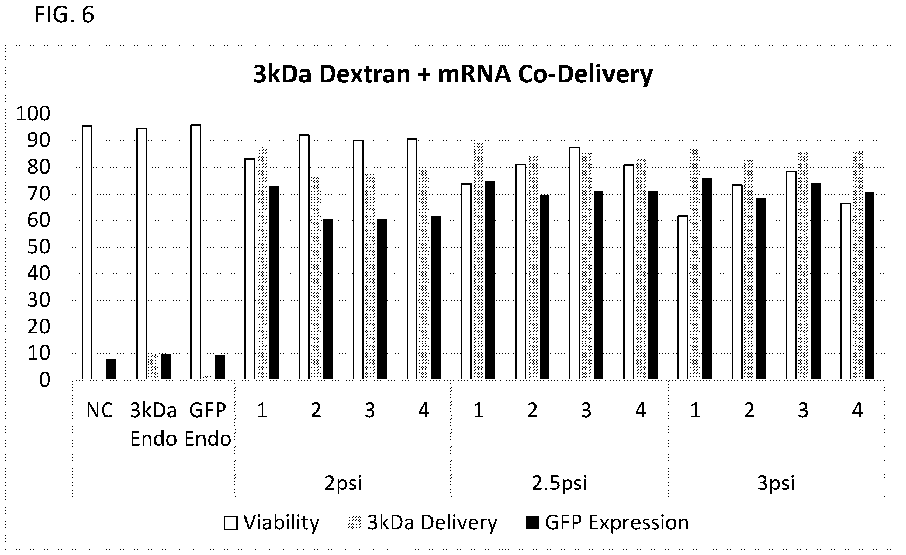

**FIG. 6** shows delivery efficiency, cell viability, and rMFI values post-delivery of 3kDa PacBlue dextran particles and EGFP mRNA to HeLa cells mediated by COTS filters at 2psi, 2.5psi, and 3psi.

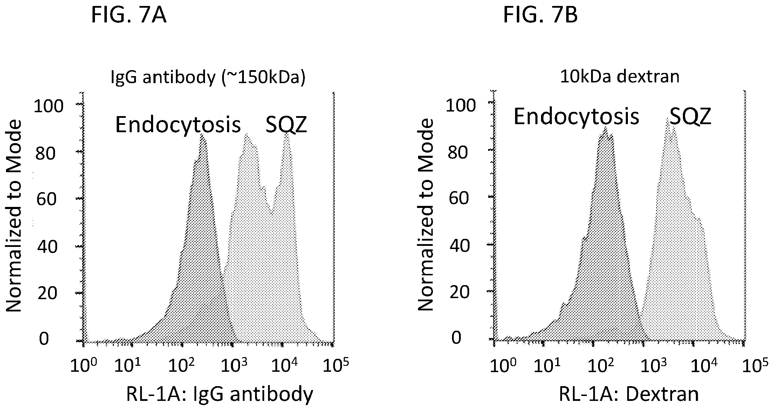

**FIGS. 7A&B** show exemplary flow cytometry histogram plots depicting fluorescence post constriction mediated delivery (SQZ) of IgG antibody (**FIG. 7A**) and dextran particles (**FIG. 7B**) to human RBCs as compared to endocytosis controls.

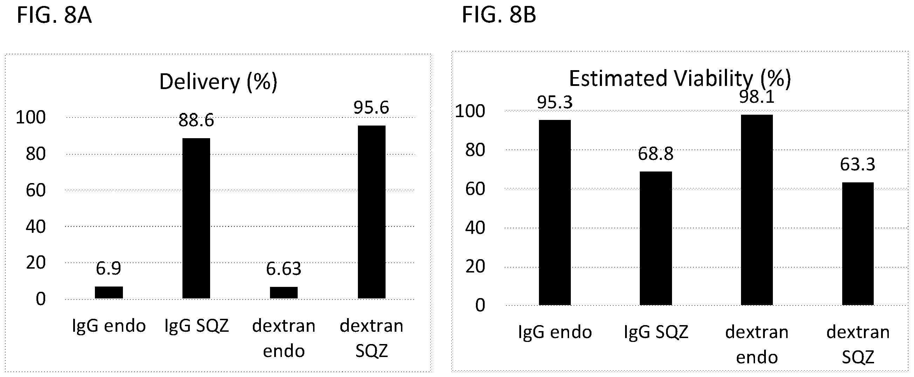

**FIG. 8A** shows delivery efficiency post constriction mediated delivery of IgG antibody and dextran particles to human RBCs. **FIG. 8B** shows estimated cell viability post constriction mediated delivery of IgG antibody and dextran particles to human RBCs.

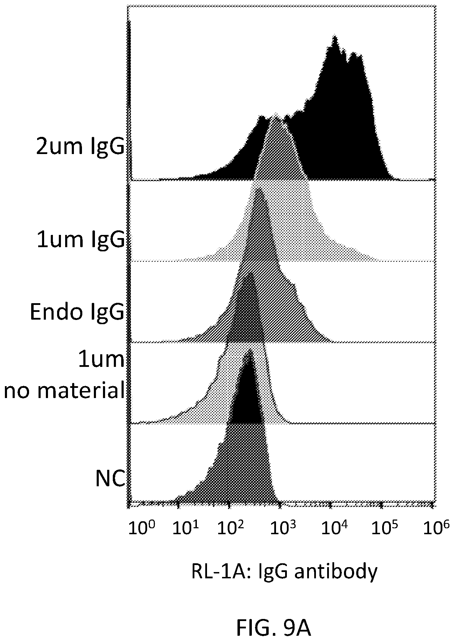

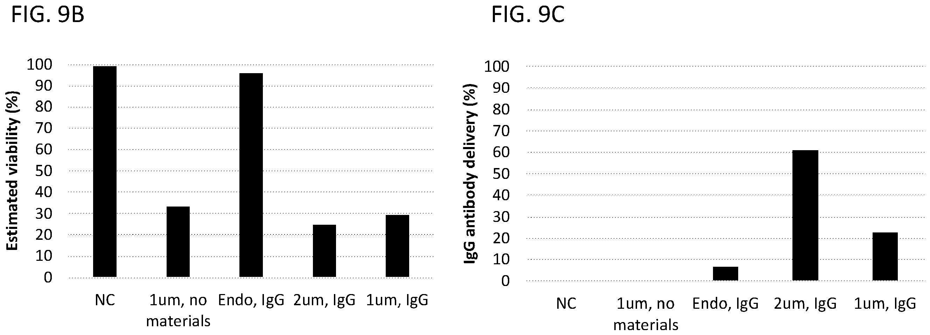

**FIG. 9A** shows exemplary flow cytometry histogram plots depicting fluorescence post constriction mediated delivery of IgG antibody to mouse RBCs. **FIG. 9B** shows estimated cell viability post constriction mediated delivery of IgG antibody to mouse RBCs. **FIG. 9C** shows delivery efficiency post constriction mediated delivery of IgG antibody to mouse RBCs.

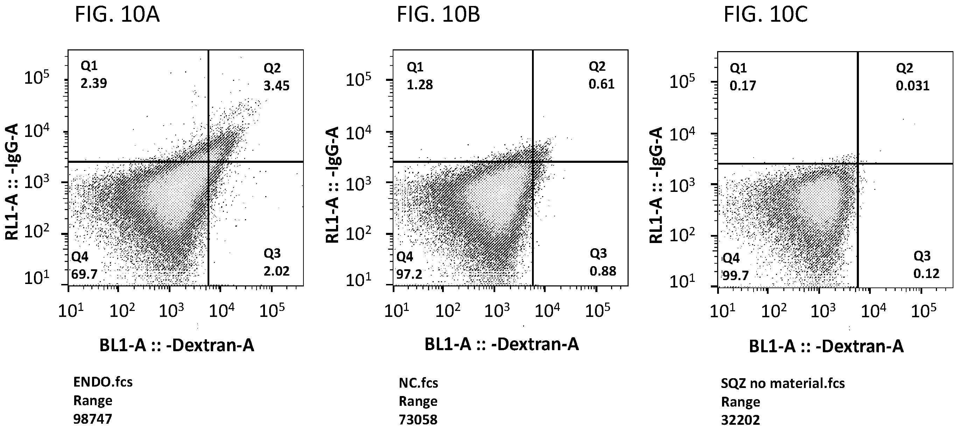

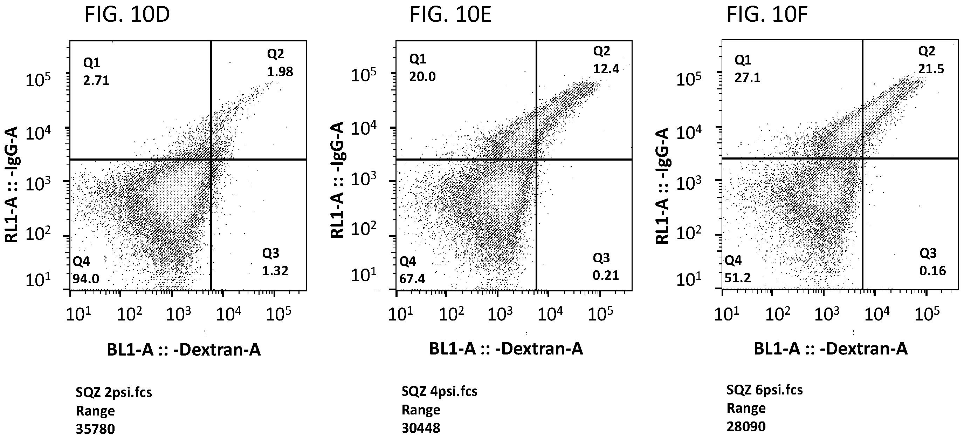

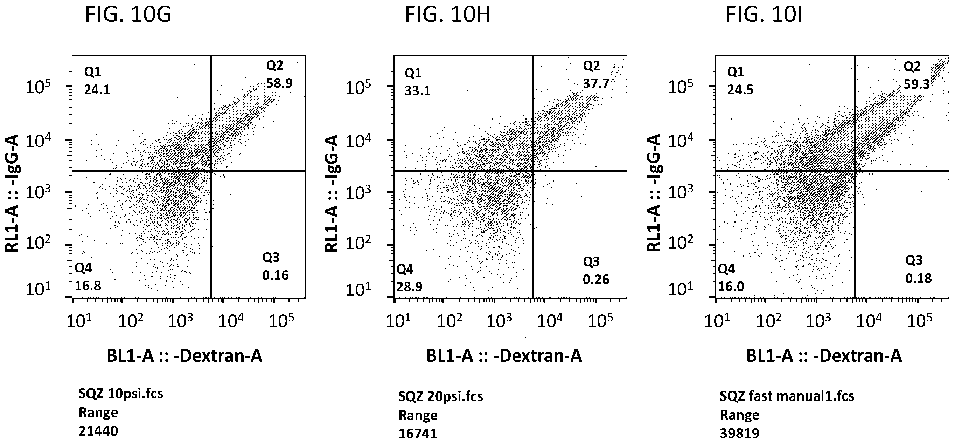

**FIGS. 10A-I** show exemplary FACS plots demonstrating delivery of dextran particles to mouse RBCs under 2psi (**FIG. 10D**), 4psi (**FIG. 10E**), 6psi (**FIG. 10F**), 10psi (**FIG. 10G**), 20psi (**FIG. 10H**), or using manual syringe pressure (**FIG. 101**) as compared to the endocytosis (**FIG. 10A**), negative control (**FIG. 10B**), and no material control (**FIG**. **10C**).

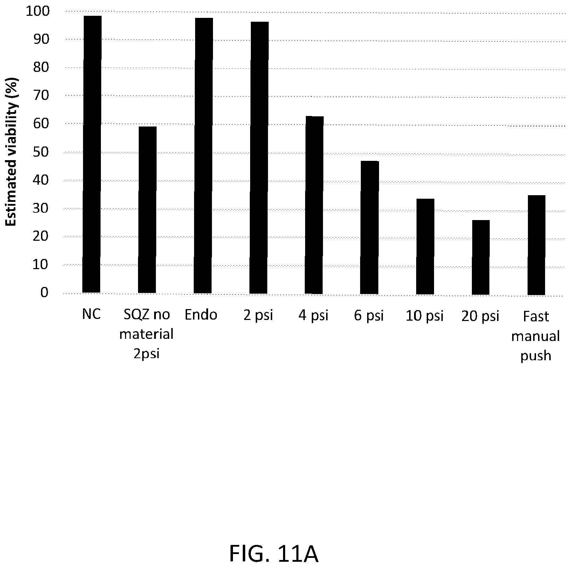

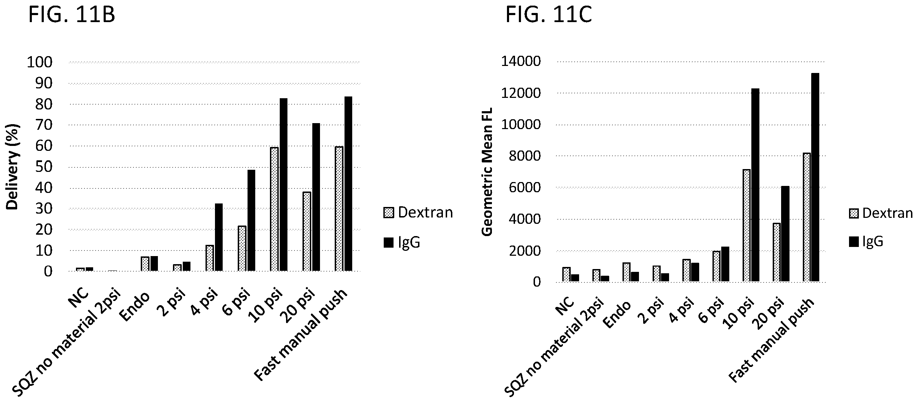

**FIG. 11A** shows estimated cell viability post constriction mediated delivery of dextran particles to mouse RBCs. **FIG. 11B** shows delivery efficiency post constriction mediated delivery of IgG antibody or dextran particles to mouse RBCs. **FIG. 11C** shows geometric mean fluorescence post constriction mediated delivery of IgG antibody or dextran particles to mouse RBCs.

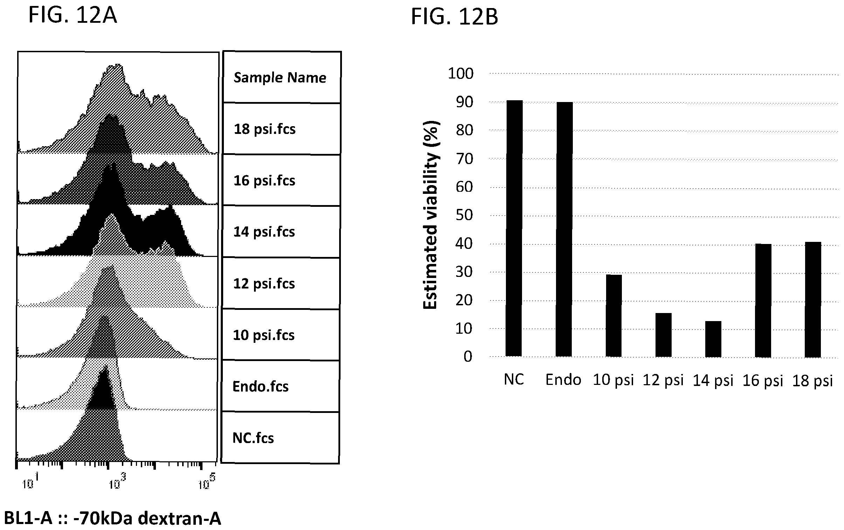

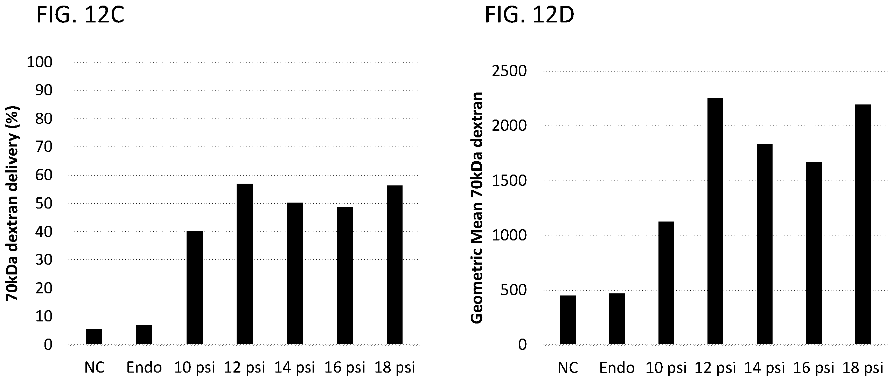

**FIG. 12A** shows exemplary flow cytometry histogram plots depicting fluorescence post constriction mediated delivery of dextran particles to mouse RBCs. **FIG. 12B** shows estimated cell viability post constriction mediated delivery of dextran particles to mouse RBCs. **FIG. 12C** shows delivery efficiency post constriction mediated delivery of dextran particles to mouse RBCs. **FIG. 12D** shows geometric mean fluorescence post constriction mediated delivery of dextran particles to mouse RBCs.

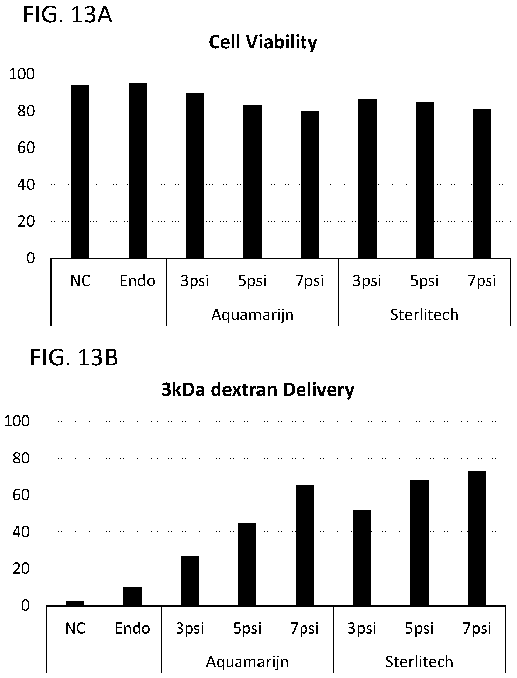

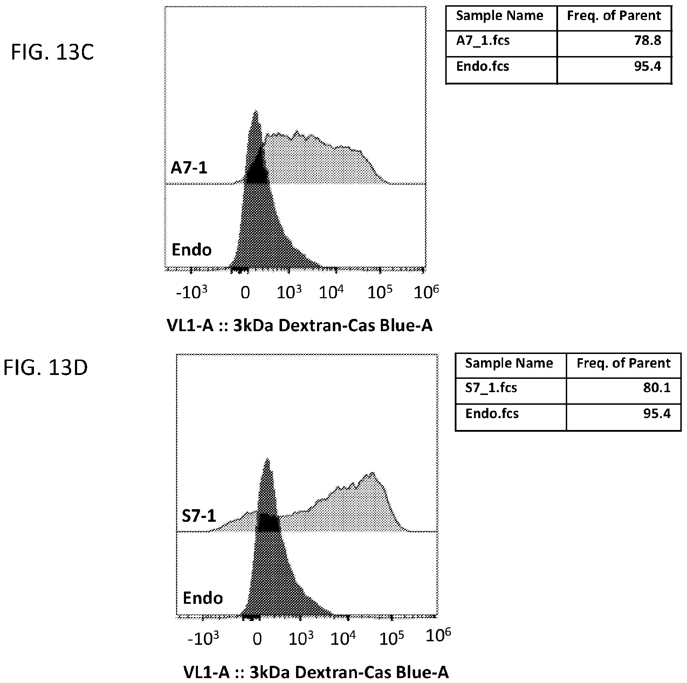

**FIG. 13A** shows cell viability post microsieve mediated delivery of dextran particles to HeLa cells. **FIG. 13B** shows delivery efficiency post microsieve mediated delivery of dextran particles to HeLa cells. **FIGS. 13C** and **FIG. 13D** shows exemplary flow cytometry histogram plots depicting fluorescence post constriction mediated delivery of dextran particles to HeLa cells using an AQUAMARIJN microsieve (**FIG. 13C**) or a STERLITECH^{™} microsieve (**FIG. 13D**).

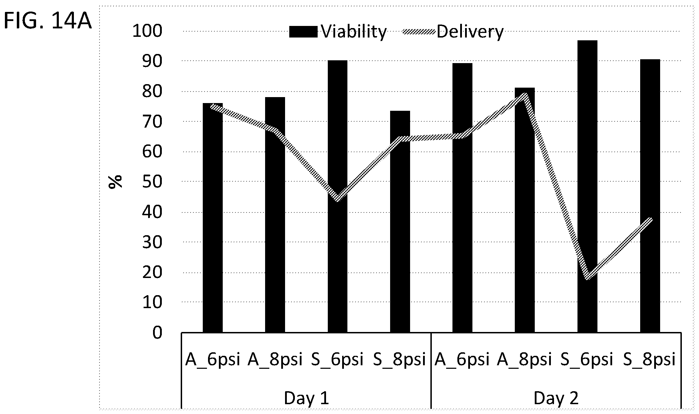

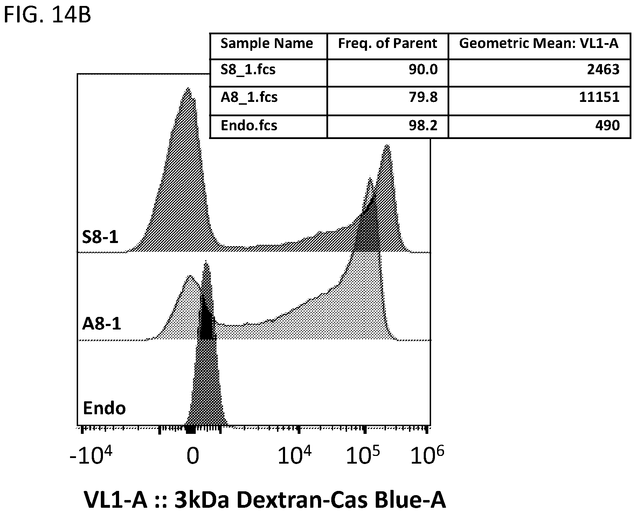

**FIG. 14A** shows cell viability and delivery efficiency post microsieve mediated delivery of dextran particles to T cells. **FIGS. 14B** shows exemplary flow cytometry histogram plots depicting fluorescence post constriction mediated delivery of dextran particles to T cells using an AQUAMARIJN microsieve.

### DETAILED DESCRIPTION

Certain aspects of the disclosures described herein are based upon the surprising discovery that intracellular delivery of compounds can be achieved by passing a cell suspension through a surface containing pores. Certain aspects of the present disclosure relate to methods for delivering a compound into a cell, the methods including passing a cell suspension through a surface containing pores, wherein said pores deform the cell thereby causing a perturbation of the cell such that the compound enters the cell, wherein said cell suspension is contacted with the compound. Certain aspects of the present disclosure relate to a device for delivering a compound into a cell, said device including a surface containing pores, wherein said pores are configured such that a cell suspended in a solution can pass through, wherein said pores deform the cell thereby causing a perturbation of the cell such that the compound enters the cell. In some embodiments, the surface is a filter. In some embodiments, the surface is a membrane. In some embodiments, the surface is a microsieve.

### I. GENERAL TECHNIQUES

The techniques and procedures described or referenced herein arc generally well understood and commonly employed using conventional methodology by those skilled in the art, such as, for example, the widely utilized methodologies described in Molecular Cloning: A Laboratory Manual (Sambrook et al., 4th ed., Cold Spring Harbor Laboratory Press, Cold Spring Harbor, N.Y., 2012); Current Protocols in Molecular Biology (DOI: 10.1002/0471142727); the series Methods in Enzymology (Academic Press, Inc.); PCR 2: A Practical Approach (M.J. MacPherson, B.D. Hames and G.R. Taylor eds., 1995); Antibodies, A Laboratory Manual (E. A. Greenfield, eds., 2013); Culture of Animal Cells: A Manual of Basic Technique and Specialized Applications (R.I. Freshney, 6th ed., J. Wiley and Sons, 2010); Oligonucleotides and Analogues (F. Eckstein, ed., 1992); Methods in Molecular Biology, Humana Press; Cell Biology: A Laboratory Handbook (J.E. Celis, ed., Academic Press, 2005); Introduction to Cell and Tissue Culture (J.P. Mather and P.E. Roberts, Springer, 2013); Cell and Tissue Culture: Laboratory Procedures (A. Doyle, J.B. Griffiths, and D.G. Newell, eds., J. Wiley and Sons, 1993-8); Handbook of Experimental Immunology (D.M. Weir and C.C. Blackwell, eds., 1996); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (DOI: 10.1002/0471142735); Short Protocols in Molecular Biology (Ausubel et al., eds., J. Wiley and Sons, 2002); Janeway's Immunobiology (K. Murphy and C. Weaver, Garland Science, 2016); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane, Cold Spring Harbor Laboratory Press, 1999); Making and Using Antibodies: A Practical Handbook (G.C. Howard and M.R. Kaser, eds., CRC Press, 2013); The Antibodies Vol. 1-7 (M. Zanetti and J. D. Capra, eds., Harwood Academic Publishers, 1995-2007); and Cancer: Principles and Practice of Oncology (V.T. DeVita et al., eds., J.B. Lippincott Company, 2011).

### II. DEFINITIONS

For purposes of interpreting this specification, the following definitions will apply and whenever appropriate, terms used in the singular will also include the plural and vice versa. In the event that any definition set forth below conflicts with any document, the definition set forth shall control.

As used herein, the singular form "a", "an", and "the" includes plural references unless indicated otherwise.

It is understood that aspects and embodiments of the disclosure described herein include "comprising," "consisting," and "consisting essentially of' aspects and embodiments.

For all compositions described herein, and all methods using a composition described herein, the compositions can either comprise the listed components or steps, or can "consist essentially of" the listed components or steps. When a composition is described as "consisting essentially of' the listed components, the composition contains the components listed, and may contain other components which do not substantially affect the methods disclosed, but do not contain any other components which substantially affect the methods disclosed other than those components expressly listed; or, if the composition does contain extra components other than those listed which substantially affect the methods disclosed, the composition does not contain a sufficient concentration or amount of the extra components to substantially affect the methods disclosed. When a method is described as "consisting essentially of' the listed steps, the method contains the steps listed, and may contain other steps that do not substantially affect the methods disclosed, but the method does not contain any other steps which substantially affect the methods disclosed other than those steps expressly listed. As a non-limiting specific example, when a composition is described as 'consisting essentially of' a component, the composition may additionally contain any amount of pharmaceutically acceptable carriers, vehicles, or diluents and other such components which do not substantially affect the methods disclosed.

The term "about" as used herein refers to the usual error range for the respective value readily known to the skilled person in this technical field. Reference to "about" a value or parameter herein includes (and describes) embodiments that are directed to that value or parameter per se.

The term "pore" as used herein refers to an opening, including without limitation, a hole, tear, cavity, aperture, break, gap, or perforation within a material. In some examples, (where indicated) the term refers to a pore within a surface of the present disclosure. In other examples, (where indicated) a pore can refer to a pore in a cell membrane.

The term "membrane" as used herein refers to a selective barrier or sheet containing pores. The term includes a pliable sheetlike structure that acts as a boundary or lining. In some examples, the term refers to a surface or filter containing pores. This term is distinct from the term "cell membrane".

The term "filter" as used herein refers to a porous article that allows selective passage through the pores. In some examples the term refers to a surface or membrane containing pores.

The term "heterogeneous" as used herein refers to something which is mixed or not uniform in structure or composition. In some examples the term refers to pores having varied sizes, shapes or distributions within a given surface.

The term "homogeneous" as used herein refers to something which is consistent or uniform in structure or composition throughout. In some examples the term refers to pores having consistent sizes, shapes, or distribution within a given surface.

The term "heterologous" as used herein refers to a molecule which is derived from a different organism. In some examples the term refers to a nucleic acid or protein which is not normally found or expressed within the given organism.

The term "homologous" as used herein refers to a molecule which is derived from the same organism. In some examples the term refers to a nucleic acid or protein which is normally found or expressed within the given organism.

The term "polynucleotide" or "nucleic acid" as used herein refers to a polymeric form of nucleotides of any length, either ribonucleotides or deoxyribonucleotides. Thus, this term includes, but is not limited to, single-, double- or multi-stranded DNA or RNA, genomic DNA, cDNA, DNA-RNA hybrids, or a polymer comprising purine and pyrimidine bases, or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidates and thus can be an oligodeoxynucleoside phosphoramidate (P-NH2) or a mixed phosphoramidate- phosphodiester oligomer. In addition, a double-stranded polynucleotide can be obtained from the single stranded polynucleotide product of chemical synthesis either by synthesizing the complementary strand and annealing the strands under appropriate conditions, or by synthesizing the complementary strand de novo using a DNA polymerase with an appropriate primer.

The terms "polypeptide" and "protein" are used interchangeably to refer to a polymer of amino acid residues, and are not limited to a minimum length. Such polymers of amino acid residues may contain natural or non-natural amino acid residues, and include, but are not limited to, peptides, oligopeptides, dimers, trimers, and multimers of amino acid residues. Both full-length proteins and fragments thereof are encompassed by the definition. The terms also include post-expression modifications of the polypeptide, for example, glycosylation, sialylation, acetylation, phosphorylation, and the like. Furthermore, for purposes of the present invention, a "polypeptide" refers to a protein which includes modifications, such as deletions, additions, and substitutions (generally conservative in nature), to the native sequence, as long as the protein maintains the desired activity. These modifications may be deliberate, as through site-directed mutagenesis, or may be accidental, such as through mutations of hosts which produce the proteins or errors due to PCR amplification.

For any of the structural and functional characteristics described herein, methods of determining these characteristics are known in the art.

### III. SURFACE HAVING PORES

In certain aspects, the present disclosure relates to methods for delivering a compound into a cell comprising the steps of passing a cell suspension through a surface containing pores, wherein the pores deform the cell, causing a perturbation of the cell, and contacting the cell suspension with the compound; e.g., before, during or after the cells in the suspension pass through the pores, wherein the compound enters the cell. The surfaces as disclosed herein can be made of any one of a number of materials and take any one of a number of forms. In some embodiments, the surface is a membrane. In some embodiments, the surface is a filter. Filters and membranes are normally used to separate a material from a solution, thereby obtaining a retentate. In embodiments where the surface is a filter or a membrane, the filter or membrane is alternatively used for delivering a compound into a cell by passing a cell suspension through the filter or membrane pores, thereby causing a perturbation of the cell such that a compound enters the cell. In some embodiments, the surface is a STERLITECH^{™} polycarbonate filter (PCT8013100). In some embodiments, the filter is a tangential flow filter. In some embodiments, the surface is a sponge or sponge-like matrix. In some embodiments, the surface is a matrix. In some embodiments, the surface is a microsieve. In some embodiments, the surface is not a net.

In some embodiments the surface is a tortuous path surface. In some embodiments, the tortuous path surface comprises cellulose acetate. In some embodiments, the surface comprises a material selected from, without limitation, synthetic or natural polymers, polycarbonate, silicon, glass, metal, alloy, cellulose nitrate, silver, cellulose acetate, nylon, polyester, polyethersulfone, Polyacrylonitrile (PAN), polypropylene, PVDF, polytetrafluorethylene, mixed cellulose ester, porcelain, graphite, and ceramic.

The surface of the present disclosure may be manufactured using any technique known in the art, including, without limitation, etching, track-etching, lithography, laser ablation, injection molding, stamping, micro-hole punching, polymeric-sponge, direct foaming, extrusion, and hot embossing. Etching involves the process of using a chemical, such as a strong acid, to cut into a metal surface in a desired pattern. Track etched membranes are formed by bombarding a solid film with particles that form tracks of damaged material through the film. The film is then subjected to a chemical agent that selectively etches the damaged tracks to create perforations through the film. The cross-sectional widths of the pores can be controlled by the incubation time of the etchant on the film. Lithography can include microlithography, nanolithography, and x-ray lithography. In lithography, a lithographic apparatus applies a desired pattern onto a target portion of the substrate. For example, in x-ray lithography, x-rays are used to transfer a pattern from a mask to a light-sensitive chemical photoresist on the desired substrate. In photolithography, light is used to transfer the desired pattern from a photomask to a light-sensitive photoresist on the substrate. Subsequent chemical application is used to engrave the pattern into the substrate material beneath the photoresist. Laser ablation is the process of removing material from a solid surface by irradiating with a laser beam, while injection molding involves injecting a material into a desired mold, where it then cools and hardens into the desired structure. Stamping and micro-hole punching methods utilize tools to cut or imprint desired forms into the substrate material. To produce ceramic filter membranes, the polymeric-sponge method involves saturating a polymeric sponge with a ceramic slurry, which is then burned out to leave a porous ceramic. In the direct foaming method, a chemical mixture containing the desired ceramic component and organic materials is treated to evolve a gas. Bubbles are then produced in the material, causing it to foam. The resulting porous ceramic material is then dried and fired. To produce a honeycomb or cellular structure, a plastic-forming method called extrusion is used, where a mixture of ceramic powder plus additives is forced through a shaped die. Hot embossing involves the stamping of a pattern into a polymer softened by raising the temperature of the polymer just above its glass transition temperature.

In some embodiments, the surface is a nanostructure membrane. In some embodiments, phase change is used to produce nanostructured membranes. Phase change methods include, without limitation, precipitation from the vapor phase, dry-wet phase inversion, and thermally induced phase separation. In the precipitation from the vapor phase method, a cast polymer solution composed of polymer and solvent is introduced into a nonsolvent vapor environment saturated with solvent vapor. The saturated solvent vapor suppresses the evaporation of solvent from the film. The nonsolvent molecules subsequently diffuse into the film, leading to polymer setting. In the dry-wet phase inversion method, a polymer solution composed of polymer and solvent is prepared. The solution is cast on a suitable surface and after partial evaporation of the solvent, the cast film is immersed in a gelatin. Then, the nonsolvent diffuses into the polymer solution film through the thin solid layer while the solvent diffuses out, creating a porous membrane. In the thermally induced phase separation method, a polymer is mixed with a solvent at a high temperature and the polymer solution is cast into a film. When the solution is cooled, it enters into an immiscible area due to the loss of solvency.

In some embodiments, the surface is a block copolymer. Block copolymers are composed of two or more blocks of different polymerized monomers linked by covalent bonds. Block copolymer components can microphase separate to form periodic nanostructures. In this process, due to incompatibility between the blocks, block copopolymers undergo phase separation, creating nanometer-sized structures.

In some embodiments, the surface is a microsieve. In some examples, microsieves are used in cell separation, CTC isolation or droplet emulsification techniques. In some embodiments, pores of a microsieve are generated by photolithography on a ceramic substrate with a silicon support. In some embodiments, the microsieve is made of polycarbonate. Microsieves may be available from Aquamarijn or Sterlitech. In some embodiments, the microsieve has a pore size ranging from about 4 µm to about 10 µm. In some embodiments, the microsieve has a porosity of greater than about any of 5%, 8%, 10%, 15%, 20%, 25%, 30%, 35%, 40%, 45%, or greater than about 50%.

The surface disclosed herein can have any shape known in the art; e.g. a 3-dimensional shape. The 2-dimensional shape of the surface can be, without limitation, circular, elliptical, round, square, star-shaped, triangular, polygonal, pentagonal, hexagonal, heptagonal, or octagonal. In some embodiments, the surface is round in shape. In some embodiments, the surface 3-dimensional shape is cylindrical, conical, or cuboidal.

The terms "pore size" and "pore cross-sectional width" are used interchangeably, and as used herein refer to the smallest cross-sectional width across the pore. In some embodiments, the pore is circular or roughly circular and the pore size or pore cross sectional width. In some embodiments, the pore is polygonal in shape (e.g., square, rectangular, pentagonal, hexagonal, etc.) and the pore size or pore cross sectional width is the smallest width of the polygon. One skilled in the art would understand that a triangular pore may not have a width, but rather, is described in terms of bases and heights. In some embodiments, the pore size or pore cross sectional width of a triangular pore is the smallest height of the triangle (smallest distance between a base and its opposite angle).

The surface can have various cross-sectional widths and thicknesses. In some embodiments, the surface cross-sectional width is between about 1mm and about 3m or any cross-sectional width or range of cross-sectional widths therebetween. In some embodiments, the surface cross-sectional width is between any one of about 1mm to about 750mm, about 1mm to about 500mm, about 1mm to about 250mm, about 1mm to about 100mm, about 1mm to about 50mm, about 1mm to about 25mm, about 1mm to about 10mm, about 1mm to about 5mm, or about 1mm to about 2.5mm. In some embodiments, the surface cross-sectional width is between any one of about 5mm to about 1m, about 10mm to about 1m, about 25mm to about 1m, about 50mm to about 1m, about 100mm to about 1m, about 250mm to about 1m, about 500mm to about 1m, or about 750mm to about 1m.

In some embodiments, the surface has a defined thickness. In some embodiments, the surface thickness is uniform. In some embodiments, the surface thickness is variable. For example, in some embodiments, portions of the surface are thicker or thinner than other portions of the surface. In some embodiments, the surface thickness varies by about 1-90% or any percentage or range of percentages therebetween. In some embodiments, the surface thickness varies by about any one of about 1% to about 90%, about 1% to about 80%, about 1% to about 70%, about 1% to about 60%, about 1% to about 50%, about 1% to about 40%, about 1% to about 30%, about 1% to about 20%, about 1% to about 10%, or about 1% to about 5%. In some embodiments, the surface thickness varies by any one of about 5% to about 90%, about 10% to about 90%, about 20% to about 90%, about 30% to about 90%, about 40% to about 90%, about 50% to about 90%, about 60% to about 90%, about 70% to about 90%, or about 80% to about 90%. In some embodiments, the surface is between about 0.01µm to about 5mm thick or any thickness or range of thicknesses therebetween. In some embodiments, the surface is between about 0.01µm to about 5mm, about 0.01µm to about 2.5mm, about 0.01µm to about 1mm, about 0.01µm to about 750µm, about 0.01µm to about 500µm, about 0.01µm to about 250µm, about 0.01µm to about 100µm, about 0.01µm to about 90µm, about 0.01µm to about 80µm, about 0.01µm to about 70µm, about 0.01µm to about 60µm, about 0.01µm to about 50µm, about 0.01µm to about 40µm, about 0.01µm to about 30µm, about 0.01um to about 20µm, about 0.01µm to about 10µm, about 0.01µm to about 5µm , about 0.01µm to about 1µm, about 0.01µm to about 0.5µm, about 0.01µm to about 0.1µm, or about 0.01µm to about 0.05µm thick. In some embodiments, the surface is between about 0.01µm to about 5mm, about 0.05µm to about 5mm, about 0.1µm to about 5mm, about 0.5µm to about 5mm, about 1µm to about 5mm, about 5µm to about 5mm, about 10µm to about 5mm, about 20um to about 5mm, about 30µm to about 5mm, about 40µm to about 5mm, about 50µm to about 5mm, about 60µm to about 5mm, about 70µm to about 5mm, about 80µm to about 5mm, about 90µm to about 5mm, about 100µm to about 5mm, about 250µm to about 5mm, about 500µm to about 5mm, about 750µm to about 5mm, about 1mm to about 5mm, or about 2.5mm to about 5mm thick. In some embodiments, the surface is about 10µm thick.

In some embodiments, the flow rate through the surface is between about 0.001 mL/cm²/sec to about 100 L/cm²/sec or any rate or range of rates therebetween. In some embodiments, the flow rate is between about 0.001 mL/cm²/sec to about 75 L/cm²/sec, about 0.001 mL/cm ²/sec to about 50 L/cm²/sec, about 0.001 mL/cm²/sec to about 25 L/cm²/sec, about 0.001 mL/cm ²/sec to about 10 L/cm²/sec, about 0.001 mL/cm²/sec to about 7.5 L/cm²/sec, about 0.001 mL/cm²/sec to about 5.0 L/cm²/sec, about 0.001 mL/cm²/sec to about 2.5 L/cm ²/sec, about 0.001 mL/cm²/sec to about 1 L/cm²/sec, about 0.001 mL/cm²/sec to about 0.1 L/cm²/sec, about 0.001 mL/cm²/sec to about 75 mL/cm²/sec, about 0.001 mL/cm ²/sec to about 50 mL/cm²/sec, about 0.001 mL/cm²/sec to about 25 mL/cm²/sec, about 0.001 mL/cm²/sec to about 10 mL/cm²/sec, about 0.001 mL/cm²/sec to about 1 mL/cm²/sec, about 0.001 mL/cm ²/sec to about 0.1 mL/cm²/sec, or about 0.001 mL/cm²/sec to about 0.01 mL/cm²/sec. In some embodiments, the flow rate is between about 0.001 mL/cm²/sec to about 100 L/cm²/sec, about 0.01 mL/cm²/sec to about 100 L/cm²/sec, about 0.1mL/cm²/sec to about 100 L/cm²/sec, about 1 mL/cm²/sec to about 100 L/cm²/sec, about 10 mL/cm²/sec to about 100 L/cm²/sec, about 50 mL/cm²/sec to about 100 L/cm²/sec, about 0.1 L/cm²/sec to about 100 L/cm²/sec, about 0.5 L/cm²/sec to about 100 L/cm²/sec, about 1 L/cm²/sec to about 100 L/cm²/sec, about 2.5 L/cm²/sec to about 100 L/cm²/sec, about 5 L/cm²/sec to about 100 L/cm²/sec, about 7.5 L/cm²/sec to about 100 L/cm²/sec, about 10 L/cm²/sec to about 100 L/cm²/sec, about 25 L/cm²/sec to about 100 L/cm²/sec, about 50 L/cm²/sec to about 100 L/cm²/sec, or about 75 L/cm²/sec to about 100 L/cm²/sec.

The cross-sectional width of the pores is related to the type of cell to be treated. In some embodiments, the pore size is a function of the diameter of the cell to be treated. In some embodiments, the pore size is such that a cell is perturbed upon passing through the pore. In some embodiments, the pore size is less than the diameter of the cell. In some embodiments, the pore size is about 20-99% of the diameter of the cell. In some embodiments, the pore size is about any one of about 20%, 30%, 40%, 50%, 60%, 70%, 80%, 90% or 99% of the diameter of the cell or any value therebetween. Optimal pore size can vary based upon the application and/or cell type. Many cells are between about 5-15 µm in diameter, e.g. dendritic cells are 7-8 µm in diameter. For example, the cross-sectional width of the pore is less than about any of 4.5, 5, 5.5, 6, or 6.5 µm for processing of single cells. In another example, the cross-sectional width of the pores for processing of human eggs is between about 6.2 µm and about 8.4 µm, although larger and smaller pores are possible (diameter of a human ovum is approximately 120 µm). In some embodiments, clusters of cells (e.g., embryos) are processed using a pore cross-sectional width of between about 12 µm and about 17 µm, given that the cluster of cells is not disrupted when passing through the pore. In some embodiments, the pore cross-sectional width ranges from about 0.01µm to about 300µm or any size or range of sizes therebetween. In some embodiments, the pore cross-sectional width size ranges from about 0.01µm to about 250µm, about 0.01µm to about 200µm, about 0.01µm to about 150µm, about 0.01µm to about 100µm, about 0.01µm to about 50µm, or about 0.01µm to about 25µm. In some embodiments, the pore cross-sectional width ranges from about 1µm to about 300µm, about 25µm to about 300µm, about 50µm to about 300µm, about 100µm to about 300µm, about 150µm to about 300µm, about 200µm to about 300µm or about 250µm to about 300µm. In some embodiments, the pore cross-sectional width ranges from about 0.01 to about 35µm. In some embodiments, the pore cross-sectional width is at or less than any of about 0.4µm, about 5µm, about 10µm, about 12µm, or about 14µm. In some embodiments, the pore cross-sectional width is at least about 1µm, 2µm, 3µm, 4µm, or 5µm. In some embodiments, the pore cross-sectional width is at or less than about 200µm. In some embodiments, the pores are heterogeneous in cross-sectional width or homogeneous in cross-sectional width across a given surface. In some embodiments, the heterogeneous pore cross-sectional widths vary from 10-20% or any percentage or range of percentages therebetween. In some embodiments, the pore deforms the cell to about any one of about 5%, 10%, 20%, 30%, 40%, 50%, 60%, 70%, 80%, 90% or 99% of the diameter of the cell or any value therebetween. In some embodiments, the size of the cell is the size of the cell in suspension.

In some embodiments, the cross-sectional area of the pore is a function of the cross-sectional area of the cell. In some embodiments, the two-dimensional shape of the pore is circular, elliptical, square, rectangular, star-shaped, triangular, polygonal, pentagonal, hexagonal, heptagonal, or octagonal and the cross-sectional area of the pore is a function of the cross-sectional area of the cell. In some embodiments, the pore cross-sectional area is at least about 1µm², 4µm², 9µm², 16µm², 25µm², 50µm², 100µm², 150µm², 200µm², 250µm² 500µm² or 1000µm². In some embodiments, the pores are heterogeneous in cross-sectional area or homogeneous in cross-sectional area across a given surface. In some embodiments, the heterogeneous pore cross-sectional area varies from 10-20% or any percentage or range of percentages therebetween. In some embodiments, the pore deforms the cell to about any one of about 5%, 10%, 20%, 30%, 40%, 50%, 60%, 70%, 80%, 90% or 99% of the cross-sectional area of the cell or any value therebetween. In some embodiments, the size of the cell is the size of the cell in suspension.

The entrances and exits of the pore passage may have a variety of angles. The pore angle can be selected to minimize clogging of the pore while cells are passing through. For example, the angle of the entrance or exit portion can be between about 0 and about 90 degrees. In some embodiments, the pores have identical entrance and exit angles. In some embodiments, the pores have different entrance and exit angles. In some embodiments, the pore edge is smooth, e.g. rounded or curved. A smooth pore edge has a continuous, flat, and even surface without bumps, ridges, or uneven parts. In some embodiments, the pore edge is sharp. A sharp pore edge has a thin edge that is pointed or at an acute angle. In some embodiments, the pore passage is straight. A straight pore passage does not contain curves, bends, angles, or other irregularities. In some embodiments, the pore passage is curved. A curved pore passage is bent or deviates from a straight line. In some embodiments, the pore passage has multiple curves, e.g. about 2, 3, 4, 5, 6, 7, 8, 9, 10 or more curves.

The pores can have any shape known in the art, including a 2-dimensional or 3-dimensional shape. The pore shape (e.g., the cross-sectional shape) can be, without limitation, circular, elliptical, round, rectangular, square, star-shaped, triangular, polygonal, pentagonal, hexagonal, heptagonal, and octagonal. In some embodiments, the cross-section of the pore is round in shape. In some embodiments, the 3-dimensional shape of the pore is cylindrical or conical. In some embodiments, the pore has a fluted entrance and exit shape. In some embodiments, the pore shape is homogenous (i.e. consistent or regular) among pores within a given surface. In some embodiments, the pore shape is heterogeneous (i.e. mixed or varied) among pores within a given surface.

The surfaces described herein can have a range of total pore numbers. In some embodiments, the pores encompass about 10-80% of the total surface area. In some embodiments, the surface contains about 1.0×10³ to about 1.0×10³⁰ total pores or any number or range of numbers therebetween. For example, the surface may contain at least about any of 1.0×10³, 1.0×10⁴, 1.0×10⁵, 1.0×10⁶, 1.0×10⁷, 1.0×10⁸, 1.0×10⁹, 1.0×10¹⁰, 1.0×10¹⁵, 1.0×10²⁰, 1.0×10²⁵, 1.0×10³⁰, or more total pores. In some embodiments, the surface comprises between about 10 and about 1.0×10¹⁵ pores per mm² surface area. For example, the surface may contain about 1.0×10² to about 1.0×10¹⁵, about 1.0×10³ to about 1.0×10¹⁵, about 1.0×10⁵ to about 1.0×10¹⁵, about 1.0×10⁷ to about 1.0×10¹⁵, about 1.0×10¹⁰ to about 1.0×10¹⁵, about 1.0×10¹² to about 1.0×10¹⁵ pores per mm² surface area. In some embodiments, the surface may contain about 10 to about 1.0×10¹⁵ , about 10 to about 1.0×10¹², about 10 to about 1.0×10¹⁰, about 10 to about 1.0×10⁷, about 10 to about 1.0×10⁵, about 10 to about 1.0×10³, or about 10 to about 1.0x10² pores per mm² surface area. In some embodiments, a surface with a 13mm radius comprises about 6.0×10⁵ pores.

The pores can be distributed in numerous ways within a given surface. In some embodiments, the pores are distributed in parallel within a given surface. In some embodiments, surfaces with pores are stacked on top of each other. In one such example, the pores are distributed side-by-side in the same direction and are the same distance apart within a given surface. In some embodiments, the pore distribution is ordered or homogeneous. In one such example, the pores are distributed in a regular, systematic pattern or are the same distance apart within a given surface. In some embodiments, the pore distribution is random or heterogeneous. In one such example, the pores are distributed in an irregular, disordered pattern or are different distances apart within a given surface. In some embodiments, the pores in the surface are arranged in an irregular pattern. In some embodiments, the pores in the surface are heterogeneous in size. In some embodiments, the pores in the surface are heterogeneous in shape. In some embodiments, the pores in the surface are arranged in an irregular pattern and are heterogeneous in size. In some embodiments, the pores in the surface are arranged in an irregular pattern and are heterogeneous in shape. In some embodiments, the pores in the surface are heterogeneous in size and heterogeneous in shape. In some embodiments, the pores in the surface are arranged in an irregular pattern, are heterogeneous in size, and are heterogeneous in shape.

In some embodiments, multiple surfaces are distributed in series. The multiple surfaces can be homogeneous or heterogeneous in surface size, shape, and/or roughness. The multiple surfaces can further contain pores with homogeneous or heterogeneous pore size, shape, and/or number, thereby enabling the simultaneous delivery of a range of compounds into different cell types. In some embodiments, the multiple surfaces are stacked. In some embodiments, the cell suspension passes through multiple surfaces.

In some embodiments, an individual pore has a uniform width dimension (i.e. constant width along the length of the pore passage). In some embodiments, an individual pore has a variable width (i.e. increasing or decreasing width along the length of the pore passage). In some embodiments, pores within a given surface have the same individual pore depths. In some embodiments, pores within a given surface have different individual pore depths. In some embodiments, the pores are immediately adjacent to each other. In some embodiments, the pores are separated from each other by a distance. In some embodiments, the pores are separated from each other by a distance of about 0.001µm to about 30mm or any distance or range of distances therebetween. For example, the pores may be separate from each other by a distance of between any one of about 0.001µm to 30mm, about 0.01µm to about 30mm, about 0.05µm to about 30mm, about 0.1µm to about 30mm, about 0.5µm to about 30mm, about 1µm to about 30mm, about 5µm to about 30mm, about 10µm to about 30mm, about 50µm to about 30mm, about 100µm to about 30mm, about 250µm to about 30mm, about 500µm to about 30mm, about 1mm to about 30mm, about 10mm to about 30mm, or about 20mm to about 30mm. In some embodiments, the pores may be separated from each other by a distance of between any one of about 0.001µm to 0.01µm, about 0.001µm to about 0.05µm, about 0.001µm to about 0.1µm, about 0.001µm to about 0.5µm, about 0.001µm to about 1µm, about 0.001µm to about 5µm, about 0.001µm to about 10µm, about 0.001µm to about 50µm, about 0.001µm to about 100µm, about 0.001µm to about 250µm, about 0.001µm to about 500µm, about 0.001µm to about 1mm, about 0.001µm to about 10mm, or about 0.001µm to about 20mm.

In some embodiments, the surface is coated with a material. The material can be selected from any material known in the art, including, without limitation, Teflon, an adhesive coating, surfactants, anticoagulants such as heparin, EDTA, citrate, and oxalate, proteins, adhesion molecules, antibodies, factors that modulate cellular function, nucleic acids, lipids, carbohydrates, complexes such as lipid-carbohydrate complexes, or transmembrane proteins. In some embodiments, the surface is coated with polyvinylpyrrolidone. In some embodiments, the material is covalently attached to the surface. In some embodiments, the material is non-covalently attached to the surface. In some embodiments, the surface molecules are released at the cells pass through the pores.

In some embodiments, the surface has modified chemical properties. In some embodiments, the surface is hydrophilic. In some embodiments, the surface is hydrophobic. In some embodiments, the surface is charged. In some embodiments, the surface is positively and/or negatively charged. In some embodiments, the surface can be positively charged in some regions and negatively charged in other regions. In some embodiments, the surface has an overall positive or overall negative charge. In some embodiments, the surface can be any one of smooth, electropolished, rough, or plasma treated. In some embodiments, the surface comprises a zwitterion or dipolar compound.

### IV. CELL SUSPENSIONS

In certain aspects, the present disclosure relates to passing a cell suspension through a surface containing pores. In some embodiments, the cell suspension comprises animal cells. In some embodiments, the cell suspension comprises frog, chicken, insect, or nematode cells. In some embodiments, the cell suspension comprises mammalian cells. In some embodiments, the cell is a mouse, dog, cat, horse, rat, goat or rabbit cell. In some embodiments, the cell is a human cell.

In some embodiments, the cell suspension comprises a cell comprising a cell wall. In some embodiments, the cell is a plant, yeast, fungal, algal, or bacterial cell. In some embodiments, the cell is a plant cell. In some embodiments, the plant cell is a crop, model, ornamental, vegetable, leguminous, conifer, or grass plant cell. In some embodiments, the cell is a yeast cell. In some embodiments, the yeast cell is a *Candida* or *Saccharomyces* cell. In some embodiments, the cell is a fungal cell. In some embodiments, the fungal cell is an *Aspergillus* or *Penicillium* cell. In some embodiments, the cell is an algal cell. In some embodiments, the algal cell is a *Chlamydomonas*, *Dunaliella*, or *Chlorella* cell. In some embodiments, the cell suspension comprises bacterial cells. In some embodiments, the bacterial cell is a gram-positive bacterial cell. Gram-positive bacteria have a cell wall comprising a thick peptidoglycan layer. In some embodiments, the bacterial cell is a gram-negative bacterial cell. Gram-negative bacterial have a cell wall comprising a thin peptidoglycan layer between an inner cytoplasmic cell membrane and an outer membrane. In some embodiments, the bacterial cell is a *Streptococcus*, *Escherichia*, *Enterobacter*, *Bacillus*, *Pseudomonas*, *Klebsiella*, or *Salmonella* cell.

The cell suspension may be a mixed or purified population of cells. In some embodiments, the cell suspension is a mixed cell population, such as whole blood, lymph, and/or peripheral blood mononuclear cells (PBMCs). In some embodiments, the cell suspension is a purified cell population. In some embodiments, the cell is a primary cell or a cell line cell. In some embodiments, the cell is a blood cell. In some embodiments, the blood cell is an immune cell. In some embodiments, the immune cell is a lymphocyte. In some embodiments, the immune cell is a T cell, B cell, natural killer (NK) cell, dendritic cell (DC), NKT cell, mast cell, monocyte, macrophage, basophil, eosinophil, neutrophil, or DC2.4 dendritic cell. In some embodiments, the immune cell is a primary human T cell. In some embodiments, the blood cell is a red blood cell. In some embodiments, the cell is a cancer cell. In some embodiments, the cancer cell is a cancer cell line cell, such as a HeLa cell. In some embodiments, the cancer cell is a tumor cell. In some embodiments, the cancer cell is a circulating tumor cell (CTC). In some embodiments, the cell is a stem cell. Exemplary stem cells include, without limitation, induced pluripotent stem cells (iPSCs), embryonic stem cells (ESCs), liver stem cells, cardiac stem cells, neural stem cells, and hematopoietic stem cells. In some embodiments, the cell is a fibroblast cell, such as a primary fibroblast or newborn human foreskin fibroblast (Nuff cell). In some embodiments, the cell is an immortalized cell line cell, such as a HEK293 cell or a CHO cell. In some embodiments, the cell is a skin cell. In some embodiments, the cell is a reproductive cell such as an oocyte, ovum, or zygote. In some embodiments, the cell is a neuron. In some embodiments, the cell is a cluster of cells, such as an embryo, given that the cluster of cells is not disrupted when passing through the pore.

The composition of the cell suspension (e.g., osmolarity, salt concentration, serum content, cell concentration, pH, etc.) can impact delivery of the compound. In some embodiments, the suspension comprises whole blood. Alternatively, the cell suspension is a mixture of cells in a physiological saline solution or physiological medium other than blood. In some embodiments, the cell suspension comprises an aqueous solution. In some embodiments, the aqueous solution comprises cell culture medium, PBS, salts, sugars, growth factors, animal derived products, bulking materials, surfactants, lubricants, vitamins, proteins, chelators, and/or an agent that impacts actin polymerization. In some embodiments, the cell culture medium is DMEM, OptiMEM, IMDM, RPMI, or X-VIVO. Additionally, solution buffer can include one or more lubricants (pluronics or other surfactants) that can be designed to reduce or eliminate clogging of the surface and improve cell viability. Exemplary surfactants include, without limitation, poloxamer, polysorbates, sugars such as mannitol, animal derived serum, and albumin protein.

In some configurations with certain types of cells, the cells can be incubated in one or more solutions that aid in the delivery of the compound to the interior of the cell. In some embodiments, the aqueous solution comprises an agent that impacts actin polymerization. In some embodiments, the agent that impacts actin polymerization is Latrunculin A, Cytochalasin, and/or Colchicine. For example, the cells can be incubated in a depolymerization solution such as Lantrunculin A (0.lµg/ml) for 1 hour prior to delivery to depolymerize the actin cytoskeleton. As an additional example, the cells can be incubated in 10µM Colchicine (Sigma) for 2 hours prior to delivery to depolymerize the microtubule network.

The viscosity of the cell suspension can also impact the methods disclosed herein. In some embodiments, the viscosity of the cell suspension ranges from about 8.9×10⁻⁴Pa·s to about 4.0×10⁻³Pa·s or any value or range of values therebetween. In some embodiments, the viscosity ranges between any one of about 8.9×10⁻⁴ Pa·s to about 4.0 ×10⁻³ Pa·s, about 8.9×10⁻⁴ Pa·s to about 3.0 ×10⁻³ Pa·s, about 8.9×10⁻⁴ Pa·s to about 2.0 ×10⁻³ Pa·s, or about 8.9×10⁻³ Pa·s to about 1.0 ×10⁻³ Pa·s. In some embodiments, the viscosity ranges between any one of about 0.89 cP to about 4.0 cP, about 0.89 cP to about 3.0 cP, about 0.89 cP to about 2.0 cP, or about 0.89 cP to about 1.0 cP. In some embodiments, a shear thinning effect is observed, in which the viscosity of the cell suspension decreases under conditions of shear strain. Viscosity can be measured by any method known in the art, including without limitation, viscometers, such as a glass capillary viscometer, or rheometers. A viscometer measures viscosity under one flow condition, while a rheometer is used to measure viscosities which vary with flow conditions. In some embodiments, the viscosity is measured for a shear thinning solution such as blood. In some embodiments, the viscosity is measured between about 0°C and about 45°C. For example, the viscosity is measured at room temperature (e.g., about 20°C), physiological temperature (e.g., about 37°C), higher than physiological temperature (e.g., greater than about 37°C to 45°C or more), reduced temperature (e.g., about 0°C to about 4°C), or temperatures between these exemplary temperatures.

### V. COMPOUNDS TO DELIVER

In certain aspects, the present disclosure relates to methods for delivering a compound into a cell. In some embodiments, the compound is a single compound. In some embodiments, the compound is a mixture of compounds. In some embodiments, the compound comprises a nucleic acid. In some embodiments, the compound is a nucleic acid. Exemplary nucleic acids include, without limitation, recombinant nucleic acids, DNA, recombinant DNA, cDNA, genomic DNA, RNA, siRNA, mRNA, saRNA, miRNA, IncRNA, tRNA, shRNA, self-amplifying mRNA, and peptide nucleic acids. In some embodiments, the nucleic acid is homologous to a nucleic acid in the cell. In some embodiments, the nucleic acid is heterologous to a nucleic acid in the cell. In some embodiments, the nucleic acid comprises a transposon, and optionally a sequence encoding a transposase. In some embodiments, the compound is a plasmid. In some embodiments, the nucleic acid is a therapeutic nucleic acid. In some embodiments, the nucleic acid encodes a therapeutic polypeptide. In some embodiments the nucleic acid encodes a reporter or a selectable marker. Exemplary reporter markers include, without limitation, green fluorescent protein (GFP), red fluorescent protein (RFP), auquorin, beta-galactosidase, Uroporphyrinogen (urogen) III methyltransferase (UMT), and luciferase. Exemplary selectable markers include, without limitation, Blasticidin, G418/Geneticin, Hygromycin B, Puromycin, Zeocin, Adenine Phosphoribosyltransferase, and thymidine kinase.

In some embodiments, the compound comprises a protein-nucleic acid complex. In some embodiments, the compound is a protein-nucleic acid complex. In some embodiments, protein-nucleic acid complexes, such as clustered regularly interspaced short palindromic repeats (CRISPR)-Cas9, are used in genome editing applications. These complexes contain sequence-specific DNA-binding domains in combination with nonspecific DNA cleavage nucleases. These complexes enable targeted genome editing, including adding, disrupting, or changing the sequence of a specific gene. In some embodiments, a disabled CRISPR is used to block or induce transcription of a target gene. In some embodiments, the compound contains a Cas9 protein and a guide RNA or donor DNA. In some embodiments, the compound includes a nucleic acid encoding for a Cas9 protein and a guide RNA or donor DNA. In some embodiments, the compound includes a transposase protein and a nucleic acid comprising a transposon.

In some embodiments, the compound comprises a protein or polypeptide. In some embodiments, the compound is a protein or polypeptide. In some embodiments, the protein or polypeptide is a therapeutic protein, antibody, fusion protein, antigen, synthetic protein, reporter marker, or selectable marker. In some embodiments, the protein is a gene-editing protein or nuclease such as a zinc-finger nuclease (ZFN), transcription activator-like effector nuclease (TALEN), mega nuclease, CRE recombinase, FLP recombinase, R recombinase, integrase, or transposase. In some embodiments, the fusion proteins can include, without limitation, chimeric protein drugs such as antibody drug conjugates or recombinant fusion proteins such as proteins tagged with GST or streptavidin. In some embodiments, the compound is a transcription factor. Exemplary transcription factors include, without limitation, Oct5, Sox2, c-Myc, Klf-4, T-bet, GATA3, FoxP3, and RORyt.

In some embodiments, the compound comprises an antigen. In some embodiments, the compound is an antigen. An antigen is a substance that stimulates a specific immune response, such as a cell or antibody-mediated immune response. Antigens bind to receptors expressed by immune cells, such as T cell receptors (TCRs), which are specific to a particular antigen. Antigen-receptor binding subsequently triggers intracellular signaling pathways that lead to downstream immune effector pathways, such as cell activation, cytokine production, cell migration, cytotoxic factor secretion, and antibody production. In some embodiments, antigens are derived from foreign sources, such as bacteria, fungi, viruses, or allergens. In some embodiments, antigens are derived from internal sources, such as cancer cells or self-proteins (i.e. self-antigens). Self-antigens are antigens present on an organism's own cells. Self-antigens do not normally stimulate an immune response, but may in the context of autoimmune diseases, such as Type I Diabetes or Rheumatoid Arthritis. In some embodiments, the antigen is a neoantigen. Neoantigens are antigens that are absent from the normal human genome, but are created within oncogenic cells as a result of tumor-specific DNA modifications that result in the formation of novel protein sequences.

In some embodiments the protein or polypeptide is a reporter or a selectable marker. Exemplary reporter markers include, without limitation, green fluorescent protein (GFP), red fluorescent protein (RFP), auquorin, beta-galactosidase, Uroporphyrinogen (urogen) III methyltransferase (UMT), and luciferase. Exemplary selectable markers include, without limitation, Blasticidin, G418/Geneticin, Hygromycin B, Puromycin, Zeocin, Adenine Phosphoribosyltransferase, and thymidine kinase.

In some embodiments, the compound comprises an antibody. In some embodiments, the compound is an antibody. In some embodiments, the antibody is a full length antibody or an antibody fragment. Antibodies for use in the present disclosure include, without limitation, human or humanized antibodies, antibody variants, labeled antibodies, antibody fragments such as Fab or F(ab)₂ fragments, single-domain antibodies, single-chain antibodies, multi-specific antibodies, antibody fusion proteins, and immunoadhesins. The antibodies may be any isotype known in the art, including IgA, IgG, IgE, IgD, or IgM.

In some embodiments, the compound comprises a small molecule. In some embodiments, the compound is a small molecule. Exemplary small molecules include, without limitation, fluorescent markers, dyes, pharmaceutical agents, metabolites, adjuvants, or radionucleotides. In some embodiments, the pharmaceutical agent is a therapeutic drug and/or cytotoxic agent. In some embodiments, the adjuvant includes, without limitation, CpG, oligodeoxynucleotide, R848, lipopolysaccharide (LPS), rhIL-2, anti-CD40 or CD40L, IL-12, cyclic di-nucleotides, and stimulator of interferon genes (STING) agonists.

In some embodiments, the compound comprises a nanoparticle. Examples of nanoparticles include gold nanoparticles, quantum dots, carbon nanotubes, nanoshells, dendrimers, and liposomes. In some embodiments, the nanoparticle contains a therapeutic molecule. In some embodiments, the nanoparticle contains a nucleic acid, such as mRNA. In some embodiments, the nanoparticle contains a label, such as a fluorescent or radioactive label.

In some embodiments, the compound comprises a chimeric antigen receptor (CAR). In some embodiments, the compound is a chimeric antigen receptor (CAR). In some embodiments, the CAR is a fusion of an extracellular recognition domain (e.g., an antigen-binding domain), a transmembrane domain, and one or more intracellular signaling domains. Upon antigen engagement, the intracellular signaling portion of the CAR can initiate an activation-related response in an immune cell, such are the release of cytokines or cytolytic molecules. In some embodiments, the CAR is a chimeric T-cell antigen receptor. In some embodiments, the CAR contains an antigen-binding domain specific to a tumor antigen. In some embodiments, the CAR antigen-binding domain is a single-chain antibody variable fragment (scFv).

In some embodiments, the compound comprises a fluorescently tagged molecule. In some embodiments, the compound is a fluorescently tagged molecule, such as a molecule tagged with a fluorochrome such as pacific blue, Alexa 288, Cy5, or cascade blue. In some embodiments, the compound is a radionucleotide, dextran particle, magnetic bead, or impermeable dye. In some embodiments, the compound is a 3kDa dextran particle labeled with PacBlue. In some embodiments, the compound is a lOkDa dextran particles labeled with Alexa488. In some embodiments, the compound is a small molecule fluorophore tagged protein. In some embodiments, the compound is a small molecule tagged with Alexa647.

In some embodiments, the compound comprises a virus or virus-like particle. In some embodiments, the compound is a virus or virus-like particle. In some embodiments, the virus is a retrovirus. In some embodiments, the virus is a therapeutic virus. In some embodiments, the virus is an oncolytic virus. In some embodiments, the virus or virus-like particle contains nucleic acid encoding a therapeutic molecule, such as a therapeutic polypeptide.

In some embodiments, the compound to deliver is purified. In some embodiments, the compound is at least about 60% by weight (dry weight) the compound of interest. In some embodiments, the purified compound is at least about 75%, 90%, or 99% the compound of interest. In some embodiments, the purified compound is at least about 90%, 91%, 92%, 93%, 94%, 95%, 98%, 99%, or 100% (w/w) the compound of interest. Purity is determined by any known methods, including, without limitation, column chromatography, thin layer chromatography, HPLC analysis, NMR, mass spectrometry, or SDS-PAGE. Purified DNA or RNA is defined as DNA or RNA that is free of exogenous nucleic acids, carbohydrates, and lipids.

### VI. CELL PERTURBATIONS

In certain aspects, the present disclosure relates to passing a cell suspension through a surface containing pores, wherein the pores deform the cell, causing a perturbation of the cell. The deformation can be caused by, for example, pressure induced by mechanical strain and/or shear forces. The perturbation in the cell is a breach in the cell that allows material from outside the cell to move into the cell (e.g., a hole, tear, cavity, aperture, pore, break, gap, perforation). In some embodiments, the perturbation is a perturbation within the cell membrane. In some embodiments, the perturbation is transient. In some embodiments, the cell perturbation lasts from about 1.0×10⁻⁹ seconds to about 2 hours, or any time or range of times therebetween. In some embodiments, the cell perturbation lasts for about 1.0×10⁻⁹ second to about 1 second, about 1 second to about 1 minute, or about 1 minute to about 1 hour. In some embodiments, the cell perturbation lasts for between any one of about 1.0×10⁻⁹ to about 1.0×10⁻¹, about 1.0×10⁻⁹ to about 1.0×10⁻², about 1.0×10⁻⁹ to about 1.0×10⁻³, about 1.0×10⁻⁹ to about 1.0×10⁻⁴, about 1.0×10⁻⁹ to about 1.0×10⁻⁵, about 1.0×10⁻⁹ to about 1.0×10⁻⁶, about 1.0×10⁻⁹ to about 1.0×10⁻⁷, or about 1.0×10⁻⁹ to about 1.0×10⁻⁸ seconds. In some embodiment, the cell perturbation lasts for any one of about 1.0×10⁻⁸ to about 1.0×10⁻¹, about 1.0×10⁻⁷ to about 1.0×10⁻¹, about 1.0×10⁻⁶ to about 1.0×10⁻¹, about 1.0×10⁻⁵ to about 1.0×10⁻¹, about 1.0×10⁻⁴ to about 1.0×10⁻¹, about 1.0×10⁻³ to about 1.0×10⁻¹, or about 1.0×10⁻² to about 1.0×10⁻¹ seconds. The cell perturbations (e.g., pores or holes) created by the methods described herein are not formed as a result of assembly of protein subunits to form a multimeric pore structure such as that created by complement or bacterial hemolysins.

As the cell passes through the pore of the surface, the deformation temporarily imparts injury to the cell membrane that causes passive diffusion of material through the perturbation. In some embodiments, the cell is only deformed for brief period of time, on the order of 100 µs to minimize the chance of activating apoptotic pathways through cell signaling mechanisms, although other durations are possible (e.g., ranging from nanoseconds to hours). In some embodiments, the cell is deformed for about 1.0×10⁻⁹ seconds to about 2 hours, or any time or range of times therebetween. In some embodiments, the cell is deformed for about 1.0×10⁻⁹ second to about 1 second, about 1 second to about 1 minute, or about 1 minute to about 1 hour. In some embodiments, the cell is deformed for between any one of about 1.0×10⁻⁹ to about 1.0×10⁻¹, about 1.0×10⁻⁹ to about 1.0×10⁻², about 1.0×10⁻⁹ to about 1.0×10⁻³ , about 1.0×10⁻⁹ to about 1.0×10⁻⁴, about 1.0×10⁻⁹ to about 1.0×10⁻⁵, about 1.0×10⁻⁹ to about 1.0×10⁻⁶, about 1.0×10⁻⁹ to about 1.0×10⁻⁷, or about 1.0×10⁻⁹ to about 1.0×10⁻⁸ seconds. In some embodiment, the cell is deformed for any one of about 1.0×10⁻⁸ to about 1.0×10⁻¹, about 1.0×10⁻⁷ to about 1.0×10⁻¹, about 1.0×10⁻⁶ to about 1.0×10⁻¹, about 1.0×10⁻⁵ to about 1.0×10⁻¹, about 1.0×10⁻⁴ to about 1.0×10⁻¹, about 1.0×10⁻³ to about 1.0×10⁻¹, or about 1.0×10⁻² to about 1.0×10⁻¹ seconds. In some embodiments, deforming the cell includes deforming the cell for a time ranging from, without limitation, about 1 µs to about 750 ms, e.g., at least about 1µs, 10 µs, 50µs, 100 µs, 500 µs, or 750 µs.

In some embodiments, the passage of the compound into the cell occurs simultaneously with the cell passing through the pore and/or the perturbation of the cell. In some embodiments, passage of the compound into the cell occurs after the cell passes through the pore. In some embodiments, passage of the compound into the cell occurs on the order of minutes after the cell passes through the pore of the surface. In some embodiments, the passage of the compound into the cell occurs from about 1.0×10⁻² seconds to about 30 minutes after the cell passes through the pore. For example, the passage of the compound into the cell occurs from about 1.0×10⁻² seconds to about 1 second, about 1 second to about 1 minute, or about 1 minute to about 30 minutes after the cell passes through the pore. In some embodiments, the passage of the compound into the cell occurs about 1.0×10⁻² seconds to about 10 minutes, about 1.0×10⁻² seconds to about 5 minutes, about 1.0×10⁻² seconds to about 1 minute, about 1.0×10⁻² seconds to about 50 seconds, about 1.0×10⁻² seconds to about 10 seconds, about 1.0×10⁻² seconds to about 1 second, or about 1.0×10⁻² seconds to about 0.1 second after the cell passes through the pore. In some embodiments, the passage of the compound into the cell occurs about 1.0×10⁻¹ seconds to about 10 minutes, about 1 second to about 10 minutes, about 10 seconds to about 10 minute, about 50 seconds to about 10 minutes, about 1 minute to about 10 minutes, or about 5 minutes to about 10 minutes after the cell passes through the pore. In some embodiments, a perturbation in the cell after it passes through the pores is corrected within the order of about five minutes after the cell passes through the pore.

In some embodiments, the cell viability after passing through a pore of the surface is about 10 to about 100%. In some embodiments, the cell viability after passing through the pores is at least about 10%, 20%, 30%, 40%, 50%, 60%, 70%, 75%, 80%, 85%, 90%, 95%, or 99%. In some embodiments, the cell viability is measured from about 1.0×10⁻² seconds to about 10 days after the cell passes through the pore. For example, the cell viability is measured from about 1.0×10⁻² seconds to about 1 second, about 1 second to about 1 minute, about 1 minute to about 30 minutes, or about 30 minutes to about 2 hours after the cell passes through the pore. In some embodiments, the cell viability is measured about 1.0×10⁻² seconds to about 2 hours, about 1.0×10⁻² seconds to about 1 hour, about 1.0×10⁻² seconds to about 30 minutes, about 1.0×10⁻² seconds to about 1 minute, about 1.0×10⁻² seconds to about 30 seconds, about 1.0×10⁻² seconds to about 1 second, or about 1.0×10⁻² seconds to about 0.1 second after the cell passes through the pore. In some embodiments, the cell viability is measured about 1.5 hours to about 2 hours, about 1 hour to about 2 hours, about 30 minutes to about 2 hours, about 15 minutes to about 2 hours, about 1 minute to about 2 hours, about 30 seconds to about 2 hours, or about 1 second to about 2 hours after the cell passes through the pore. In some embodiments, the cell viability is measured about 2 hours to about 5 hours, about 5 hours to about 12 hours, about 12 hours to about 24 hours, or about 24 hours to about 10 days after the cell passes through the pore.

### VII. DELIVERY PARAMETERS

In certain aspects, the present disclosure relates to methods for delivering a compound into a cell including the steps of passing a cell suspension through a surface containing pores, wherein the pores deform the cell, causing a perturbation of the cell, and contacting the cell suspension with the compound. The cell suspension may be contacted with the compound before, concurrently, or after passing through the pore. The cells may pass through the pores suspended in a solution that includes the compound to deliver, although the compound can be added to the cell suspension after the cells pass through the pores. In some embodiments, the compound to be delivered is coated on the surface.

Several parameters can influence the delivery of the compound into the cell. For example, the dimensions of the pore, the entrance angle of the pore, the surface properties of the pores (e.g. roughness, chemical modification, hydrophilic, hydrophobic, etc.), the operating flow speeds (e.g., cell transit time to the pore), the cell concentration, the concentration of the compound in the cell suspension, and the amount of time that the cell recovers or incubates after passing through the pores can affect the passage of the delivered compound into the cell. Additional parameters influencing the delivery of the compound into the cell can include the velocity of the cell in the pore, the shear rate in the pore, the velocity component that is perpendicular to flow velocity, and time in the pore. Such parameters can be designed to control delivery of the compound. In some embodiments, the cell concentration ranges from about 10 to about 10¹² cells/ml or any concentration or range of concentrations therebetween. For example, the cell concentration ranges from about 10 to about 10¹⁰ cells/ml, about 10 to about 10⁸ cells/ml, about 10 to about 10⁶ cells/ml, about 10 to about 10⁴ cells/ml, or about 10 to about 10² cells/ml. In some embodiments, the cell concentration ranges from about 10² to about 10¹² cells/ml, about 10⁴ to about 10¹² cells/ml, about 10⁶ to about 10¹² cells/ml, about 10⁸ to about 10¹² cells/ml, or about 10¹⁰ to about 10¹² cells/ml. Delivery compound concentrations can range from about 10 ng/ml to about 1g/mL or any concentration or range of concentrations therebetween. For example, the compound concentration can range from about lOng/ml to about 500mg/ml, about lOng/ml to about 250mg/ml, about lOng/ml to about 1mg/ml, about lOng/ml to about 500µg/ml, about lOng/ml to about 250µg/ml, about lOng/ml to about 1µg/ml, about lOng/ml to about 500ng/ml, about lOng/ml to about 250ng/ml, or about lOng/ml to about 100ng/ml. In some embodiments, the compound concentration can range from about lOng/ml to about 1g/ml, about 250ng/ml to about 1g/ml, about 500ng/ml to about 1g/ml, about 1µg/ml to about 1g/ml, about 250µg/ml to about 1g/ml, about 500µg/ml to about 1g/ml, about 1mg/ml to about 1g/ml, about 250mg/ml to about 1g/ml, about 500mg/ml to about 1g/ml, or about 750mg/ml to about 1g/ml. Delivery compound concentrations can range from about 1pM to about 2M. For example, the compound concentration can range from about 1pM to about 1M, about 1pM to about 500mM, about 1pM to about 250mM, about 1pM to about 1mM, about 1pM to about 500µM, about 1pM to about 250µM, about 1pM to about 1µM, about 1pM to about 500nM, about 1pM to about 250nM, about 1pM to about 100nM, about 1pM to about 10nM, about 1pM to about 5nM, about 1pM to about 1nM, about 1pM to about 750pM, about 1pM to about 500pM, about 1pM to about 250pM, about 1pM to about 100pM, about 1pM to about 50pM, about 1pM to about 25pM, or about 1pM to about 10pM. In some embodiments, the compound concentration can range from about 5pM to about 2M, about 10pM to about 2M, about 25pM to about 2M, about 50pM to about 2M, about 100pM to about 2M, about 250pM to about 2M, about 500pM to about 2M, about 750pM to about 2M, about 1nM to about 2M, about 10nM to about 2M, about 100nM to about 2M, about 500nM to about 2M, about 1µM to about 2M, about 500µM to about 2M, about 1mM to about 2M, or about 500mM to about 2M.

The temperature used in the methods of the present disclosure can be adjusted to affect compound delivery and cell viability. In some embodiments, the method is performed between about -5°C and about 45°C. For example, the methods can be carried out at room temperature (e.g., about 20°C), physiological temperature (e.g., about 37°C), higher than physiological temperature (e.g., greater than about 37°C to 45°C or more), or reduced temperature (e.g., about -5°C to about 4°C), or temperatures between these exemplary temperatures.

Various methods can be utilized to drive the cells through the pores. For example, pressure can be applied by a pump on the entrance side (e.g., gas cylinder, or compressor), a vacuum can be applied by a vacuum pump on the exit side, capillary action can be applied through a tube, and/or the system can be gravity fed. Displacement based flow systems can also be used (e.g., syringe pump, peristaltic pump, manual syringe or pipette, pistons, etc.). In some embodiments, the cells are passed through the pores by positive pressure or negative pressure. In some embodiments, the cells are passed through the pores by constant pressure or variable pressure. In some embodiments, pressure is applied using a syringe. In some embodiments, pressure is applied using a pump. In some embodiments, the pump is a peristaltic pump. In some embodiments, pressure is applied using a vacuum. In some embodiments, cells are passed through the pores by capillary pressure. In some embodiments, the cells are passed through the pores by blood pressure. In some embodiments, the cells are passed through the pores by g-force. In some embodiments, the cells are passed through the pores under a pressure differential ranging from about 0.05psi to about 500psi or any pressure or range of pressures therebetween. In some embodiments, the cells are passed through the pores under a pressure differential ranging from between about 0.05psi to about 500psi, about 0.05psi to about 400psi, about 0.05psi to about 300psi, about 0.05psi to about 200psi, about 0.05psi to about 100psi, about 0.05psi to about 50psi, about 0.05psi to about 25psi, about 0.05psi to about lOpsi, about 0.05psi to about 5psi, or about 0.05psi to about 1psi. In some embodiments, the cells are passed through the pores under a pressure differential of at least or less than about 5psi, about 10psi, or about 20psi. In some embodiments, the cells are passed through the pores under a pressure differential of at least or less than about 2psi, about 2.5psi, or about 3psi.

In some embodiments, fluid flow directs the cells through the pores. In some embodiments, the fluid flow is turbulent flow prior to the cells passing through the pore. Turbulent flow is a fluid flow in which the velocity at a given point varies erratically in magnitude and direction. In some embodiments, the fluid flow through the pore is laminar flow. Laminar flow involves uninterrupted flow in a fluid near a solid boundary in which the direction of flow at every point remains constant. In some embodiments, the fluid flow is turbulent flow after the cells pass through the pore.

The velocity at which the cells pass through the pores can be varied. In some embodiments, the cells pass through the pores at a uniform cell speed. In some embodiments, the cells pass through the pores at a fluctuating cell speed. The cells pass through the pores at a rate of at least about 0.1 mm/s to about 5 m/s, or any rate or range of rates therebetween. In some embodiments the cells pass through the pores at a range of about 0.1 mm/s to about 5 m/s, about 1mm/s to about 5 m/s, or about 1m/s to about 5m/s. In some embodiments the cells pass through the pores at a range of about 0.1 mm/s to about 4m/s, about 0.1 mm/s to about 3m/s, about 0.1 mm/s to about 2m/s, about 0.1 mm/s to about 1m/s, about 0.1 mm/s to about 750 mm/s, about 0.1 mm/s to about 500 mm/s, about 0.1 mm/s to about 250 mm/s, or about 0.1 mm/s to about 1 mm/s. In some embodiments, the cells can pass through the pores at a rate greater than about 5 m/s. The cell throughput can vary from between less than about 1 cell/second per pore to more than about 10²⁰cells/second per pore. In some embodiments, the surface allows for high throughput cell processing, on the order of billions of cells per second or minute.

In some embodiments, the cells pass the pores in one direction. In some embodiments, the cells pass through the pores in more than one direction; for example, the cells may be forced through the pore in one direction and then forced through the pore in another direction; for example, by drawing the cells through the pore using a syringe and then expelling them through the pores. In some embodiments, the cells are passed through the surface with pores more than one time.

In some embodiments, the surface is contained within a larger module. In some embodiments, the surface is contained within a syringe, such as a plastic or glass syringe. In some embodiments, the surface is contained within a plastic filter holder. In some embodiments, the surface is contained within a pipette tip.

### VIII. APPLICATIONS

In some embodiments, a compound or mixture of compounds is delivered to a cell to produce a desired effect. In some embodiments, an antigen and/or immune stimulatory molecule is delivered to a cell in order to produce professional antigen presenting cells, e.g., dendritic cells, with improved levels of activity compared to convention methods of stimulation. In some embodiments, a mixture of antigens is delivered to a cell. In some embodiments, a dendritic cell, T cell, or B cell is passed through the surface containing pores and contacted with a solution comprising a target antigen. The cells may be contacted with the antigen prior to, during, and/or after passing through the surface containing pores. In some embodiments the target antigen is a cancer cell antigen or a mixture of cancer cell antigens. In some embodiments the antigen is an infectious disease antigen. In some embodiments the antigen is a self-protein antigen. For example, delivered antigen may be a commonly expressed protein known to be associated with a particular disease or a patient-specific antigen obtained from a biopsy. In some embodiments, the antigen presenting cells produced by the method disclosed herein contribute to increased levels of T and B-cell mediated immunity against a target antigen. Such a method could thus be employed as a means of activating the immune system in response to cancer or infections or in vaccine development. One embodiment of the current subject matter includes a system in which dendritic cells, T cells or B cells, isolated from a patient's blood, are treated by the methods of the present disclosure, ex vivo, to activate them against a particular antigen and then reintroduced into the patient's blood stream. In some embodiments, the cancer antigens are from a patient with a blood cancer, such as B cell lymphoma, or with a cancer such as melanoma or pancreatic cancer. In some embodiments, cancer antigens are delivered directly to the DC cytoplasm, thereby exploiting the MHC-I antigen presentation pathway and inducing a cytotoxic T lymphocyte (CTL) response in the patient. These activated T-cells then seek out and destroy any cancerous cells which express the target antigen. In some embodiments, the method can be implemented in a typical hospital laboratory with a minimally trained technician. In some embodiments, a patient operated treatment system can be used. In some embodiments, the method is implemented using an in-line blood treatment system, in which blood is directly diverted from a patient, passed through the surface containing pores, resulting in compound delivery to blood cells, and directly transfused back into the patient after treatment.

The methods of the present disclosure can be useful in gene therapy applications. In some embodiments, the methods of the present disclosure are used to correct a genetic disease, replace a deficient gene product, or to provide a therapeutic nucleic acid. In some embodiments, gene therapy involves delivering a nucleic acid into a cell that encodes a functional, therapeutic gene to replace a mutated gene. The gene is any gene which is recognized as useful. Representative examples include genes of mammalian origin encoding, for example, proteins or useful RNAs; viral proteins such as herpes thymidine kinase, and bacterial cholera toxin for cytotoxic therapy. In some embodiments, nucleic acids, such as DNA or RNA, are delivered to cells. Nucleic acids can be delivered into difficult-to-deliver cells such as stem, primary, and immune cells. The nucleic acid can include small nucleic acids or very large nucleic acids, such as plasmids or chromosomes. Quantitative delivery into cells of known amount of a gene construct to study the expression level of a gene of interest and its sensitivity to concentration can also readily be accomplished. In some embodiments, delivery of known amounts of DNA sequences together with known amount of enzymes that enhance DNA recombination can be used to achieve more efficient stable delivery, homologous recombination, and site-specific mutagenesis.

The methods and devices described herein can also be useful for quantitative delivery of RNA for more efficient and conclusive RNA studies. Some embodiments include delivery of small interfering RNA (siRNA) into the cytoplasm of a cell. In some embodiments, RNA can be delivered into a cell for RNA silencing without the need for liposomes. Known amounts of RNA molecules together with known amounts of dicer molecules can be delivered to achieve standardized, efficient RNA levels across multiple cell lines in different conditions. In some embodiments, mRNA can be delivered into cells to study aspects of gene expression regulated at the posttranscriptional level. In some embodiments, known amounts of labeled RNA delivered to a cell can be used to study the half-life of RNAs in cells. In some embodiments, self-amplifying RNA (saRNA) is delivered to a cell. saRNAs express a protein of interest by replicating their sequences cytoplasmically without integrating into the host genome. In some embodiments, the delivered saRNAs encode antigen(s) of interest. In some embodiments, the saRNA(s) are used to continually modify cell function, for example through expression of inhibitory, stimulatory, or anti-apoptotic proteins.

For screening, imaging, or diagnostic purposes, the method of the present disclosure can be used to label cells. In some embodiments, known amounts of tagged proteins can be delivered to study protein-protein interactions in the cellular environment. Delivery of labeled antibodies into living cells for immunostaining and fluorescence-based Western blotting can be achieved. A method of labeling a cell is carried out by passing a cell through the surface containing pores and contacting the cell with a solution comprising a detectable marker. In some embodiments, the detectable marker comprises a fluorescent molecule, a radionuclide, quantum dots, gold nanoparticles, or magnetic beads. In some embodiments, engineered nanomaterials are delivered for live cell imaging applications. In some embodiments, the compound is a small molecule tagged with a fluorescent label for use in fluorescence resonance energy transfer (FRET) assays. FRET assays can be used to measure protein interactions for drug discovery applications.

In some embodiments, targeted cell differentiation is achieved by introducing proteins, mRNA, DNA and/or growth factors to induce cell reprogramming and produce iPSCs, transgenic stem cell lines, or transgenic organisms. The methods described herein, e.g., passage of stem cells or progenitor cells such as induced pluripotent stem cells (iPSCs) through a surface containing pores can be used to deliver differentiation factors into such cells. In some embodiments, after uptake of introduced factors, the cells proceed on a differentiation pathway dictated by the introduced factor.

In some embodiments, sugars are delivered into cells to improve cryopreservation of cells, such as oocytes.

### IX. ADDITIONAL EMBODIMENTS

Any of the methods described above are carried out in vitro, ex vivo, or in vivo. For in vivo applications, the device may be implanted in a vascular lumen, e.g., an in-line stent in an artery or vein. In some embodiments, the methods are used as part of a bedside system for ex-vivo treatment of patient cells and immediate reintroduction of the cells into the patient.

In other embodiments, a combination treatment is used, e.g., the methods described herein followed by or preceded by electroporation (a type of osmotic transfection in which an electric current is used to produce temporary holes in cell membranes, allowing entry of nucleic acids or macromolecules). In some embodiments, the cell is passed through an electric field generated by at least one electrode in proximity to the surface. In some embodiments, electrodes are located on one side of the surface. In some embodiments, electrodes are located on both sides of the surface. In some embodiments, the electric field is between about 0.1kV/m to about 100MV/m, or any number or range of numbers therebetween. In some embodiments, an integrated circuit is used to provide an electrical signal to drive the electrodes. In some embodiments, the cells are exposed to the electric field for a pulse width of between about Ins to about 1s and a period of between about 100ns to about 10s or any time or range of times therebetween.

Various pretreatment and post-sort assaying techniques can also be deployed, thus enabling the development of continuous, high-throughput assays for drug screening and diagnostics. In some embodiments, the methods disclosed herein may be implemented in series with a Fluorescence Activated Cell Sorting (FACS) module. This can enable the delivery and sorting of the desired cells on the same system, in real-time. For example, the methods and devices disclosed herein can also include sorters and/or sensor modules (e.g., optical, electrical, and magnetic). In some embodiments, strainers or size sorters are placed upstream of the surface to separate cells before they pass through the surface. For example, the surface as described herein can be in line with a leukophoresis device which separates white blood cells from a sample of blood. In a further embodiment, the surfaces disclosed herein can be incorporated within liquid handlers or pipettes. In some embodiments, the surface is implemented in a bioreactor format for the culture of cells and/or manufacture of biomolecules. Cells modified by the methods of the present disclosure may be used in the bioproduction of drugs or other therapeutic molecules.

In some embodiments, the surface is shaped into a tube through which a cell suspension flows through. In some embodiments, the surface shaped into a tube contains a gradient of pore sizes along its length, allowing selective delivery to different cells types as the cell suspension flows through.

Some aspects of the present disclosure involve a cell comprising a perturbation, wherein the cell is produced by passing the cell through a surface containing pores, wherein the pores deform the cell thereby causing the perturbation such that a compound is capable of entering the cell. In some embodiments, the cell is passed through an electric field generated by at least one electrode in proximity to the surface.

### X. DEVICES AND KITS

Some aspects of the present disclosure involve a device for delivering a compound into a cell, including a surface containing pores, wherein said pores are configured such that a cell suspended in a solution can pass through, wherein said pores deform the cell thereby causing a perturbation of the cell such that the compound enters the cell. The device can include any embodiment described for the methods disclosed above, including a surface having pores, cell suspensions, cell perturbations, delivery parameters, and/or applications etc. In some embodiments, at least one electrode is in proximity to the surface to generate an electric field.

In some embodiments, the device is a TRANSWELL^{™}, or permeable support. In some embodiments the TRANSWELL^{™} can be used in highthroughput screening applications. For example, a multi-well TRANSWELL^{™} filter system can be used to screen different candidate drug compounds for a particular functional or therapeutic endpoint. In some embodiments, each well contains a different compound to be tested. Cells pass through the TRANSWELL^{™} filter pores, acquiring cell perturbations, and subsequently enter into the basolateral solution containing the compound or mixture of compounds to be delivered. These methods could be deployed as a high throughput method of screening potential therapeutic compounds to identify novel treatments or understand disease mechanisms. For example, a 96 well TRANSWELL^{™} format can be used to perform high throughput screening via the methods of the present disclosure.

In some embodiments, the device contains multiple surfaces in series. The multiple surfaces can be homogeneous or heterogeneous in size, shape, and pore dimensions and/or number, thereby enabling the simultaneous delivery of a range of compounds into different cell types.

Also provided are kits or articles of manufacture for use in the methods disclosed herein. In some embodiments, the kits comprise the compositions described herein (e.g. a surface containing pores, cell suspension, and/or compounds to deliver) in suitable packaging. Suitable packaging materials are known in the art, and include, for example, vials (such as sealed vials), vessels, ampules, bottles, jars, flexible packaging (e.g., sealed Mylar or plastic bags), and the like. These articles of manufacture may further be sterilized and/or sealed.

The present disclosure also provides kits comprising components of the methods described herein and may further comprise instruction(s) for performing said methods. The kits described herein may further include other materials, including other buffers, diluents, filters, needles, syringes, and package inserts with instructions for performing any methods described herein.

### EXAMPLES

The following examples are given for the purpose of illustrating various embodiments of the disclosure and are not meant to limit the present disclosure in any fashion. One skilled in the art will appreciate readily that the present disclosure is well adapted to carry out the objects and obtain the ends and advantages mentioned, as well as those objects, ends and advantages inherent herein.

### Example 1: Delivery of dextran particles to HeLa cells

### Introduction

In order to evaluate the filter-mediated delivery of molecules into cells, HeLa cells mixed with fluorescent dextran particles were passed through filters containing pores of defined sizes, and intracellular particle delivery was evaluated via FACS analysis.

### Materials and Methods

Polycarbonate membrane filters were obtained from STERLITECH^{™} (PCT8013100). These polycarbonate filters are generated by exposing carbonate film to charged particles in a nuclear reactor to create pores of relatively uniform pore size but random distribution. The diameter of the resultant pores ranged from 0.010µm to 35µm. The filter pores were approximately 10µm thick. Exemplary images of the polycarbonate filter and filter pores are shown in **FIGS. 1A&B****.** Filters with 8µm, 10µm, 12µm, and 14µm pore sizes were used in the studies described herein. Additional materials used in the filter delivery experiments are listed in Table 1.

**Table 1: Filter Delivery Materials**

| **Material** | **Source** |
|---|---|
| Plastic filter holder (luer lock) | Pall: P/N - 4317 |
| Syringe (luer lock) | Grainger: 19G342 |
| OptiMEM media | Life Technologies |
| PBS | Life Technologies |
| 3kDA-PacBlue Dextran | Life Technologies |
| lOkDa-Alexa 488 Dextran | Life Technologies |
| Forceps | NA |

HeLa cells were suspended in optiMEM media at 4 × 10⁶ cells/mL. 100µl of the cells were pipetted out for use as negative controls. The polycarbonate membrane filter was suspended in PBS using forceps to wet the membrane filer. The plastic filter holder was uncapped, the filter was placed onto the inside surface with the shiny-side facing upwards, and the filter holder was recapped. 200µl of cells mixed with dextran particles was added into the filter holder. Dextrans were suspended at 0.2mg/mL in 5mL of cells at 4×10⁶ cells/mL. For constant pressure delivery, a pressure regulator-fitted nitrogen gas delivery system was used. The filter flow-through was collected into a 24 well plate or FACS tube. Before FACS analysis all samples were centrifuged and washed three times at 400 rcf for 4 minutes at 4°C to remove extracellular dextrans. To prepare for FACS analysis the following steps were taken. First, FACS buffer was prepared (PBS + final concentration: 1% FBS + 2nM EDTA) and Propidium Iodide was added (1:100 dilution) immediately before use. 400 µL of FACS buffer was added per tube and the cells were resuspended. The flow cytometry forward scatter, side scatter and fluorescent channel voltages were adjusted to visualize all cell events and 10,000 events/sample were recorded. Cells mixed with dextran particles, but not passed through the filters, were used as a control for particle delivery via endocytosis. These cells had contact with dye but were not passed through the filters. All other steps were the same for the endocytosis controls. The negative control samples did not have contact with dye but were subjected to the same steps as the experimental samples.

### Results

### Delivery efficiency of dextran particles to HeLa cells through 8µm, 10µm, 12µm, and 14µm sized filter pores.

HeLa cells mixed with 3kDa PacBlue and lOkDa Alexa 488 dextran particles were passed through 8µm, 10µm, 12µm, and 14µm sized filter pores at low pressure (5psi, 10psi, or 20psi). Exemplary flow cytometry plots depicting cells post-delivery are shown in **FIGS. 2A-G****.** **FIGS. 2A-C** depict cells passed through 14µm (**FIG. 2A**), 12µm (**FIG. 2B**), and 10µm (**FIG. 2C**) sized filter pores under 5psi. **FIGS. 2E****&****G** depict cells passed through 12µm filter pores under 10psi (**FIG. 2E**) and 20psi (**FIG. 2G**). **FIGS. 2D****&****F** depict endocytosis control (**FIG. 2D**) and negative control (**FIG. 2F**) plots. Cells containing both the 3kDa and lOkDa dextran particles post-filter delivery are indicated by double positive cellular staining for PacBlue and Alexa 488 (Q2 quadrant). Delivery efficiency, as indicated by percentage of cells positive for both 3kDa and lOkDa dextran particles post-filter delivery, is shown in Table 2 below. Delivery efficiency of the dextran particles varied predictably with pore size and pressure variations, with the highest delivery (86%) observed for 8µm pores at 5psi.

**Table 2: Delivery efficiency to HeLa cells**

| **Delivery %** | **8um pore** | **10um pore** | **12um pore** | **14um pore** |
|---|---|---|---|---|
| **5psi** | 86 | 34 | 26 | 10 |
| **10psi** | 48 | 56 | 29 | - |
| **20psi** | 74 | 65 | 58 | - |
| **Endocytosis** | 5 | 2 | 2 | 2 |
| **Negative Control** | 0 | 0 | 0 | 0 |

Cell viability was measured post-filter delivery. Cell viability, as indicated by percentage of live cells post-filter delivery, is shown in Table 3 below. Cell viability for cells passed through the filters decreased with increasing pressure applied, with 5psi producing the highest cell viability for each pore size. Cell viability was decreased in cells passed through the filters compared to the endocytosis control. Cell viability varied predictably with pore size and pressure variations.

**Table 3: Viability of HeLa cells post treatment**

| **Viability %** | **8um pore** | **10um pore** | **12um pore** | **14um pore** |
|---|---|---|---|---|
| **5psi** | 47 | 74 | 95 | 78 |
| **10psi** | 34 | 64 | 92 | - |
| **20psi** | 12 | 59 | 88 | - |
| **Endocytosis** | 95 | 92 | 95 | 92 |
| **Negative Control** | 93 | 92 | 97 | 90 |

### Example 2: Delivery of dextran particles to human T cells through 5µm sized filter pores.

### Introduction

In order to evaluate the filter-mediated delivery of molecules into primary immune cells, primary human T cells mixed with fluorescent dextran particles were passed through 5µm sized filter pores and intracellular particle delivery was determined via FACS analysis. Filter-mediated delivery of dextran particles to primary human T cells under manual syringe pressure or constant pressure was compared.

### Materials and Methods

Peripheral Blood Mononuclear Cells (PBMCs) were first isolated from fresh human blood via ficoll separation. The T-cells were then separated from the PBMCs by negative selection using a magnetic column. The T cells were suspended in optiMEM media at 4×10⁶ cells/mL. 100µl of the cells were pipetted out for use as negative controls. The polycarbonate membrane filter was suspended in PBS using forceps to wet the membrane filter. The plastic filter holder was uncapped, the filter was placed onto the inside surface with the shiny-side facing upwards, and the filter holder was recapped. 200µl of cells mixed with dextran particles was added into the filter holder. Dextrans were suspended at 0.2mg/mL in 5mL of cells at 4 × 10⁶ cells/mL. For constant pressure delivery, a pressure regulator-fitted nitrogen gas delivery system was used. The filter flow-through was collected into a 24 well plate or FACS tube. Before FACS analysis all samples were centrifuged and washed three times at 400 rcf for 4 minutes at 4°C to remove extracellular dextrans. To prepare for FACS analysis the following steps were taken. First, FACS buffer was prepared (PBS + final concentration: 1% FBS + 2nM EDTA) and Propidium Iodide was added (1:100 dilution) immediately before use. 400 µL of FACS buffer was added per tube and the cells were resuspended. The flow cytometry forward scatter, side scatter and fluorescent channel voltages were adjusted to visualize all cell events and 10,000 events/sample were recorded. Cells mixed with dextran particles, but not passed through the filters, were used as a control for particle delivery via endocytosis. These cells had contact with dye but were not passed through the filters. All other steps were the same for the endocytosis controls. The negative control samples did not have contact with dye but were subjected to the same steps as the experimental samples.

### Results

Primary human T cells mixed with 3kDa PacBlue and lOkDa Alexa 488 dextran particles were passed through 5µm sized filter pores under manual syringe pressure or under a constant pressure of 5psi. 8.60% of cells that passed through under manual syringe pressure (**FIG. 3A**) and 18.9% of cells that passed through under a constant pressure of 5psi (**FIG. 3B**) contained both the 3kDa and lOkDa dextran particles post-filter delivery, as indicated by double positive cellular staining for PacBlue and Alexa 488 (Q2 quadrant). 85.4% of cells that passed through under manual syringe pressure (**FIG. 3A**) and 83.1% of cells that passed through under a constant pressure of 5psi (**FIG. 3B**) were viable post-delivery.

### Example 3: Delivery of dextran particles to HeLa cells at 2psi and 3psi

### Introduction

In order to evaluate the filter-mediated delivery of molecules into cells, HeLa cells mixed with fluorescent dextran particles were passed through filters containing pores of defined sizes, and intracellular particle delivery was evaluated via FACS analysis.

### Materials and Methods

Polycarbonate membrane filters were obtained from STERLITECH^{™}. Filters with 10µm pore sizes were used in the studies described herein. HeLa cells were suspended in OptiMEM media at 2.5×10⁶ cells/mL, and 3kDa dextran particles were added at a final concentration of 0.1mg/mL. Membrane filters and plastic filter holders were prepared as described in Example 1. 200µl of cells mixed with dextran particles were added into the filter holder. For constant pressure delivery, a pressure regulator-fitted nitrogen gas delivery system was used. The filter flow-through was collected into a 24 well plate or FACS tube. Before FACS analysis all samples were centrifuged and washed three times at 400 relative centrifugal force (rcf) for 4 minutes at 4°C to remove extracellular dextrans. To prepare for FACS analysis the following steps were taken. First, FACS buffer was prepared (PBS + final concentration: 1% FBS + 2nM EDTA) and Propidium Iodide was added (1:100 dilution) immediately before use. 400 µL of FACS buffer was added per tube and the cells were resuspended. The flow cytometry forward scatter, side scatter and fluorescent channel voltages were adjusted to visualize all cell events and 10,000 events/sample were recorded. Cells mixed with dextran particles, but not passed through the filters, were used as a control for particle delivery via endocytosis. These cells had contact with dye but were not passed through the filters. All other steps were the same for the endocytosis controls. The negative control samples did not have contact with dye but were subjected to the same steps as the experimental samples. Three filters were used at each pressure (2psi and 3psi), with three samples run through each filter for each experiment. The experiment was repeated over three separate days, resulting in a total of 27 runs at each pressure.

### Results

### Delivery efficiency of dextran particles to HeLa cells through 10µm sized filter pores at 2psi and 3psi.

HeLa cells mixed with 3kDa PacBlue dextran particles were passed through 10µm sized filter pores at 2psi and 3psi. Delivery efficiency, as indicated by percentage of cells positive for 3kDa dextran particles, cell viability, as indicated by percentage of live cells post-filter delivery, and relative mean fluorescence intensity (rMFI) via flow cytometry are shown in Table 4 (2psi) and Table 5 (3psi) below. Delivery efficiency, viability, and rMFI values were repeatable and significant across experiments. At 2psi, cell viability and delivery efficiency were about 80%. At 3psi, cell viability was about 60-70%, while delivery efficiency was about 90%,

**Table 4: Filter mediated delivery to HeLa cells at 2psi**

| **2psi** | **% Viability** | **% Delivery rMFI** | |
|---|---|---|---|
| Average | 82.8 | 79.1 | 16.6 |
| Std. Dev. | 4.7 | 7.7 | 5.5 |
| % C.V. | 5.7 | 9.7 | 32.9 |

**Table 5: Filter mediated delivery to HeLa cells at 3psi**

| **3psi** | **% Viability** | **% Delivery** | **rMFI** |
|---|---|---|---|
| Average | 64.8 | 90.1 | 21.5 |
| Std. Dev. | 5.9 | 3.2 | 4.7 |
| % C.V. | 9.0 | 3.6 | 21.7 |

### Example 4: Delivery of dextran particles to HeLa cells mediated by commercial or custom syringe filters

### Introduction

In order to evaluate the filter-mediated delivery of molecules into cells, HeLa cells mixed with fluorescent dextran particles were passed through commercial or custom syringe filters, and intracellular particle delivery was evaluated via FACS analysis.

### Materials and Methods

10µm pore size polycarbonate membrane filters with a commercial filter and holder combination (COTS filters) were obtained from STERLITECH^{™}. Custom syringe filters with 10µm pores were also used.

HeLa cells were suspended in OptiMEM media at 3×10⁶ cells/mL, and 3kDa dextran particles were added at a final concentration of 0.1mg/mL. Membrane filters and plastic filter holders were prepared as described in Example 1. 200µl of cells mixed with dextran particles were added into the filter holder. For constant pressure delivery, a pressure regulator-fitted nitrogen gas delivery system was used. The filter flow-through was collected into a 24 well plate or FACS tube. Before FACS analysis all samples were centrifuged and washed three times at 400 rcf for 4 minutes at 4°C to remove extracellular dextrans. To prepare for FACS analysis the following steps were taken. First, FACS buffer was prepared (PBS + final concentration: 1% FBS + 2nM EDTA) and Propidium Iodide was added (1:100 dilution) immediately before use. 400 µL of FACS buffer was added per tube and the cells were resuspended. The flow cytometry forward scatter, side scatter and fluorescent channel voltages were adjusted to visualize all cell events and 10,000 events/sample were recorded. Cells mixed with dextran particles, but not passed through the filters, were used as a control for particle delivery via endocytosis. These cells had contact with dye but were not passed through the filters. All other steps were the same for the endocytosis controls. The negative control samples did not have contact with dye but were subjected to the same steps as the experimental samples. One filter was used at each pressure (2psi and 3psi), with three samples run through each filter for each experiment. The experiment was repeated over two separate days, resulting in a total of 6 runs at each pressure.

### Results

### Delivery efficiency of dextran particles to HeLa cells through commercial or custom filters.

HeLa cells mixed with 3kDa PacBlue dextran particles were passed through 10µm sized filter pores at 2psi and 3psi. Results from the COTS filter and custom filter were compared. Delivery efficiency, as indicated by percentage of cells positive for 3kDa dextran particles, and cell viability, as indicated by percentage of live cells post-filter delivery, are shown in **FIG. 4**. Representative flow cytometry histogram plots demonstrating mean fluorescence intensity (MFI) values for three runs at 3psi are shown in **FIG. 5A** (COTS filter) and **FIG. 5B** (custom syringe filter). Average relative mean fluorescence intensity (rMFI) values across the runs are shown in **FIG. 5C**. Removal of the mesh insert during experiments performed on the second day did not impact delivery efficiency or cell viability. No benefit was observed as a result of the dead-volume reduction when using the custom filters as compared to the COTS filters. Overall, COTS filters and custom syringe filters resulted in comparable cell viability and delivery efficiency. Subsequent experiments were performed using the COTS filters.

### Example 5: Filter mediated delivery of EGFP mRNA to HeLa cells

### Introduction

In order to evaluate the functionality of cells after filter-mediated delivery of molecules, HeLa cells mixed with fluorescent dextran particles and EGFP mRNA were passed through a 10µm pore-size COTS filter, and intracellular particle delivery and mRNA expression were evaluated via FACS analysis.

### Materials and Methods

10µm pore sizes polycarbonate membrane filters with a commercial-off-the-shelf (COTS) filter and holder combination were obtained from STERLITECH^{™}. HeLa cells were suspended in OptiMEM media at 2×10⁶ cells/mL, 3kDa dextran particles were added at a final concentration of 0.1mg/mL, and mRNA encoding EGFP was added at a final concentration of 0.1mg/mL. Membrane filters and plastic filter holders were prepared as described in Example 1. 200µl of cells mixed with dextran particles were added into the filter holder and passed through the filter at 2psi, 2.5psi, or 3psi. For constant pressure delivery, a pressure regulator-fitted nitrogen gas delivery system was used. Flow-through was collected into a 24 well plate or FACS tube.

Before FACS analysis all samples were centrifuged and washed three times at 400 rcf for 4 minutes at 4°C to remove extracellular dextrans. To prepare for FACS analysis the following steps were taken. First, FACS buffer was prepared (PBS + final concentration: 1% FBS + 2nM EDTA) and Propidium Iodide was added (1:100 dilution) immediately before use. 400 µL of FACS buffer was added per tube and the cells were resuspended. The flow cytometry forward scatter, side scatter and fluorescent channel voltages were adjusted to visualize all cell events and 10,000 events/sample were recorded. Cells mixed with dextran particles or EGFP mRNA, but not passed through the filters, were used as controls for delivery via endocytosis. These cells had contact with dye or EGFP mRNA but were not passed through the filters. All other steps were the same for the endocytosis controls. The negative control samples did not have contact with dye or EGFP mRNA but were subjected to the same steps as the experimental samples. Four samples were run through a filter at each pressure (2psi, 2.5psi, or 3psi).

### Results

HeLa cells mixed with 3kDa PacBlue dextran particles and EGFP mRNA were passed through 10µm sized filter pores at 2psi, 2.5psi, or 3psi. Delivery efficiency, as indicated by percentage of cells positive for 3kDa dextran particles or percentage of cells expressing GFP, cell viability, as indicated by percentage of live cells post-filter delivery, and relative mean fluorescence intensity (rMFI) via flow cytometry are shown in **FIG. 6** and Tables 6-8 below. Comparable delivery efficiency and cell viability was observed at 2.5psi and 3psi, with higher delivery observed compared to 2psi. Cells retained functionality 24 hours after co-delivery of dextran particles and EGFP mRNA, as indicated by sustained viability and GFP expression.

**Table 6: Filter mediated delivery of 3kDa dextran particles and EGFP mRNA to HeLa cells at 2psi**

| **2psi** | **% Viability** | **3kDa % Delivery** | **3kDa rMFI** | **GFP Expression** | **GFP rMFI** |
|---|---|---|---|---|---|
| Average | 88.8 | 80.2 | 11.5 | 63.9 | 21.4 |
| Std. Dev. | 3.4 | 4.3 | 1.6 | 5.3 | 3.9 |
| % C.V. | 3.9 | 5.4 | 14.1 | 8.3 | 18.2 |

**Table 7: Filter mediated delivery of 3kDa dextran particles and EGFP mRNA to HeLa cells at 2.5psi**

| **2.5psi** | **% Viability** | **3kDa %Delivery** | **3kDa rMFI** | **GFP Expression** | **GFP rMFI** |
|---|---|---|---|---|---|
| Average | 80.6 | 85.3 | 12.8 | 71.4 | 21.0 |
| Std. Dev. | 4.8 | 2.1 | 0.5 | 1.9 | 1.5 |
| % C.V. | 6.0 | 2.5 | 4.2 | 2.6 | 7.3 |

**Table 8: Filter mediated delivery of 3kDa dextran particles and EGFP mRNA to HeLa cells at 3psi**

| **3psi** | **% Viability** | **3kDa % Delivery** | **3kDa rMFI** | **GFP Expression** | **GFP rMFI** |
|---|---|---|---|---|---|
| Average | 69.8 | 85.1 | 12.8 | 72.1 | 20.5 |
| Std. Dev. | 6.3 | 1.6 | 0.6 | 3.0 | 1.9 |
| % C.V. | 9.0 | 1.9 | 4.6 | 4.1 | 9.1 |

### Example 6: Constriction-mediated delivery of dextran and IgG antibody to human RBCs

### Introduction

In order to evaluate filter-mediated delivery of molecules into anucleate cells, human RBCs mixed with fluorescent dextran particles or IgG antibody were passed through syringe filters containing pores of defined sizes, and intracellular particle delivery was evaluated via FACS analysis.

### Materials and Methods

2µm diameter pore size polycarbonate membrane filters with a commercial filter and holder combination (COTS filters) were obtained from STERLITECH^{™}. Human RBCs were separated from whole blood or leukoreduction collar using a Ficoll gradient separation method and resuspended at the desired concentration (50-500M/mL) in Optimem. The polycarbonate membrane filter was suspended in Optimem using forceps to wet the membrane filer. The plastic filter holder was uncapped, the filter was placed onto the inside surface with the shiny-side facing upwards, and the filter holder was recapped. Dextran particles and IgG antibody were suspended at 0.1mg/mL. 1mL of cells mixed with dextran particles or IgG antibody was added into the filter holder at room temperature. A manual finger push on the syringe was used to drive the cells through the filter. The filter flow-through was collected into a 15ml Falcon tube or FACS tube. Before FACS analysis all samples were centrifuged and washed three times at 400 rcf for 4 minutes at 4°C to remove extracellular dextrans. To prepare for FACS analysis the following steps were taken. FACS buffer was prepared (PBS + final concentration: 1% FBS + 2mM EDTA) and 400 µL of FACS buffer was added per tube and the cells were resuspended. The flow cytometry forward scatter, side scatter and fluorescent channel voltages were adjusted to visualize all cell events and ≥ 10,000 events/sample were recorded. Cells mixed with dextran particles or IgG antibody, but not passed through the filters, were used as a control for delivery via endocytosis. These cells had contact with dye but were not passed through the filters. All other steps were the same for the endocytosis controls.

### Results

RBCs mixed with lOkDa Alexa Fluor^{®} 647 dextran particles or 150kDa IgG antibody conjugated to Alexa Fluor^{®} 647 were passed through 2µm sized filter pores. Exemplary flow cytometry histogram plots depicting fluorescence post constriction mediated delivery (SQZ) versus the endocytosis control are shown in **FIGS. 7A&B****.** Delivery efficiency, as indicated by percentage of cells positive for the dextran particles of IgG antibody post-filter delivery, is shown in **FIG. 8A****.** Delivery efficiency of IgG antibody was 88.6%, and delivery efficiency of dextran particles was 95.6% post-filter delivery.

Cell viability was measured post-filter delivery. Estimated cell viability, as indicated by percentage of cells present within the FSC and SSC gate post-filter delivery, is shown in **FIG. 8B****.** Estimated viability of cells delivered IgG antibody was 68.8%, and estimated viability of cells delivered dextran particles was 63.3% post-filter delivery.

Constriction mediated delivery of dextran particles and IgG antibody into human RBCs was achieved.

### Example 7: Constriction-mediated delivery of dextran and IgG antibody to mouse RBCs

### Introduction

In order to evaluate filter-mediated delivery of molecules into anucleate cells, mouse RBCs mixed with fluorescent dextran particles or IgG antibody were passed through syringe filters containing pores of defined sizes, and intracellular particle delivery was evaluated via FACS analysis.

### Materials and Methods

1µm and 2µm diameter pore size polycarbonate membrane filters with a commercial filter and holder combination (COTS filters) were obtained from STERLITECH^{™}. Whole blood was spun down and cells were resuspended at the desired concentration (100-500 M/ml) in Optimem. The polycarbonate membrane filter was suspended in Optimem using forceps to wet the membrane filer. The plastic filter holder was uncapped, the filter was placed onto the inside surface with the shiny-side facing upwards, and the filter holder was recapped. Dextran particles and IgG antibody were suspended at 0.1mg/mL. 1mL of cells mixed with dextran particles or IgG antibody was added into the filter holder at room temperature. A manual finger push on the syringe was used to drive the cells through the filter. Alternatively, a controlled pressure system is used to drive 200µL of cells mixed with dextran particles or IgG antibody through the filter. The filter flow-through was collected into a 15ml Falcon tube or FACS tube. Before FACS analysis all samples were centrifuged and washed three times at 1000 rfc for 10 minutes at 24°C to remove extracellular dextrans. To prepare for FACS analysis the following steps were taken. FACS buffer was prepared (PBS + final concentration: 1% FBS + 2mM EDTA) and 400 µL of FACS buffer was added per tube and the cells were resuspended. The flow cytometry forward scatter, side scatter and fluorescent channel voltages were adjusted to visualize all cell events and ≥ 10,000 events/sample were recorded. Cells mixed with dextran particles or IgG antibody, but not passed through the filters, were used as a control for delivery via endocytosis. These cells had contact with dye but were not passed through the filters. All other steps were the same for the endocytosis controls. The NC (No contact) samples did not have contact with dye but were subjected to the same steps as the experimental samples but were not passed through the filters.

### Results

Mouse RBCs mixed with IgG antibody (150kDa) were passed through 1µm and 2µm sized filter pores using manual syringe pressure. Exemplary flow cytometry histogram plots depicting fluorescence post constriction mediated delivery (SQZ) versus the endocytosis (Endo), negative (NC), and no material controls control are shown in **FIG. 9A****.** The 'no material' control did not have contact with IgG antibody but was subjected to the same steps as the experimental samples and was passed through the filter.

Cell viability was measured post-filter delivery. Estimated cell viability, as indicated by percentage of cells present within the FSC and SSC gate post-filter delivery of IgG antibody using manual syringe pressure, is shown in **FIG. 9B****.** Delivery efficiency of IgG antibody, as indicated by percentage of cells positive for IgG antibody post-filter delivery using manual syringe pressure, is shown in **FIG. 9C****.**

RBCs mixed with 70kDa Alexa Fluor^{®} 488 dextran particles or IgG antibody (150kDa) were passed through 2µm sized filter pores using a controlled pressure system under 2psi, 4psi, 6psi, 10psi, 20psi, or using manual syringe pressure. Exemplary flow cytometry histogram plots depicting fluorescence post constriction mediated delivery (SQZ) versus the endocytosis (Endo), negative (NC), and no material controls control are shown in **FIGS. 10A-I****.** Cell viability was measured post-filter delivery. Estimated cell viability, as indicated by percentage of cells present within the FSC and SSC gate post-filter delivery of dextran particles is shown in **FIG. 11A**. Delivery efficiency, as indicated by percentage of cells positive for dextran particles or IgG antibody post-filter delivery is shown in **FIG. 11B**. The geometric mean fluorescence post-filter delivery of dextran particles or IgG antibody is shown in **FIG. 11C**.

RBCs mixed with 70kDa Alexa Fluor^{®} 488 dextran particles were passed through 2µm sized filter pores using a controlled pressure system under 10psi, 12psi, 14psi, 16psi, or 18psi. Exemplary flow cytometry histogram plots depicting fluorescence post constriction mediated delivery (SQZ) versus the endocytosis (Endo), and negative (NC) controls are shown in **FIG. 12A****.** Cell viability was measured post-filter delivery. Estimated cell viability, as indicated by percentage of cells present within the FSC and SSC gate post-filter delivery of dextran particles is shown in **FIG. 12B****.** Delivery efficiency, as indicated by percentage of cells positive for dextran particles post-filter delivery is shown in **FIG. 12C****.** The geometric mean fluorescence post-filter delivery of dextran particles is shown in **FIG. 12D****.**

Constriction mediated delivery of dextran particles and IgG antibody into mouse RBCs was achieved.

### Example 8. Microsieve delivery to HeLa cells and T cells

In order to evaluate microsieve-mediated delivery of molecules, HeLa cells or T cells were mixed with fluorescent dextran particles and were passed through a microsieve, and intracellular particle delivery was evaluated via FACS analysis.

### Materials and Methods

10µm pore sizes polycarbonate microsieves from STERLITECH^{™} (porosity 8%) or 10µm pore sizes silicon/ceramic microsieves from AQUAMARIJN (porosity 36.3%) were used for HeLa cells. HeLa cells were suspended in OptiMEM media at 5 × 10⁶ cells/mL, and 3kDa dextran particles were added at a final concentration of 1mg/mL. 200µl of cells mixed with dextran particles were added to the microsieve and passed through the microsieve at 3-7psi at room temperature. Recovery was at 37°C.

Naive T cells were suspended in OptiMEM media at 10 × 10⁶ cells/mL, and 3kDa dextran particles were added at a final concentration of 1mg/mL. 200µl of cells mixed with dextran particles were passed through a 4µm filter or 4 µm microsieve at 6-8psi on ice. Recovery was at room temperature.

Before FACS analysis all samples were centrifuged and washed three times at 400 rcf for 4 minutes at 4°C to remove extracellular dextrans. To prepare for FACS analysis the following steps were taken. First, FACS buffer was prepared (PBS + final concentration: 1% FBS + 2nM EDTA) and Propidium Iodide was added (1:100 dilution) immediately before use. 400 µL of FACS buffer was added per tube and the cells were resuspended. The flow cytometry forward scatter, side scatter and fluorescent channel voltages were adjusted to visualize all cell events and 10,000 events/sample were recorded. Cells mixed with dextran particles, but not passed through the filters, were used as controls for delivery via endocytosis. These cells had contact with dye or EGFP mRNA but were not passed through the filters. All other steps were the same for the endocytosis controls. The negative control samples did not have contact with dye or EGFP mRNA but were subjected to the same steps as the experimental samples.

### Results

Results for microsieve-mediated delivery of dextran to HeLa cells is shown in **FIGS. 13A-13D****.** Cell viability is shown in **FIG. 13A** and delivery of dextran is shown in **FIG. 13B** for STERLITECH^{™} and AQUAMARIJN micosieves. Representative histograms for the AQUAMARIJN and STERLITECH^{™} micosieves are shown in **FIGS 13C** and **13D****,** respectively.

Results for microsieve-mediated delivery of dextran to T cells is shown in **FIGS. 14A** **and** **14B****.** Cell viability and delivery of dextran is shown in **FIG. 14A** for AQUAMARIJN microsieves. Representative flow cytometry histograms for the AQUAMARIJN microsieves are shown in **FIG. 14B****.** Results were comparable to filter-mediated delivery.

Results show good delivery and minimal loss in viability following microsieve delivery of dextran to HeLa cells and T cells.

All of the compositions and/or methods disclosed and claimed herein can be made and executed without undue experimentation in light of the present disclosure. While the compositions and methods of this disclosure have been described in terms of embodiments, it will be apparent to those of skill in the art that variations may be applied to the compositions and/or methods and in the steps or in the sequence of steps of the method described herein without departing from the concept, spirit and scope of the disclosure. More specifically, it will be apparent that certain agents which are both chemically and physiologically related may be substituted for the agents described herein while the same or similar results would be achieved.

## Claims

1. A method for delivering a compound into a cell, the method comprising passing a cell suspension through a surface containing pores, wherein said pores deform the cell thereby causing a perturbation of the cell such that the compound enters the cell, wherein said cell suspension is contacted with the compound, further wherein the cell suspension comprises:
(i) a mixed cell population, wherein the cell suspension is whole blood, lymph or comprises peripheral blood mononuclear cells; or
(ii) a purified primary cell population, wherein the primary cell is an immune cell, a stem cell, a tumor cell, a fibroblast, a skin cell, a neuron, or a red blood cell; further wherein the surface is a membrane and/or a filter.

2. The method of claim 1, where the cell suspension comprises peripheral blood mononuclear cells.

3. The method of claim 1, wherein the cell suspension comprises a purified primary cell population wherein the primary cell is a red blood cell.

4. The method of claim 1 wherein the purified primary cell population comprises an immune cell selected from a T cell, a B cell, a dendritic cell, a monocyte, a macrophage, an eosinophil, a basophil, a NK cell, a NKT cell, a mast cell or a neutrophil.

5. The method of any one of claims 1-4, wherein:
(i) the surface comprises polycarbonate;
(ii) the surface is manufactured using etching;
(iii) the pore cross-sectional width ranges from about 0.4 to about 35µm; or
(iv) the surface is about 0.01µm to about 5mm thick.

6. The method of any one of claims 1-5, wherein the surface is coated with a material.

7. The method of any one of claims 1-6, wherein the compound:
(i) comprises a nucleic acid;
(ii) comprises a polypeptide-nucleic acid complex;
(iii) comprises nucleic acid encoding for a Cas9 protein and a guide RNA or donor DNA;
(iv) comprises a protein or peptide
(v) is a small molecule;
(vi) is a nanoparticle;
(vii) is a chimeric antigen receptor;
(viii) is a liposome; or
(ix) is a fluorescently tagged molecule.

8. The method of any one of claims 1-7, wherein said cell suspension is contacted with the compound before, concurrently, or after passing through the pore and/or the cells are passed through the pores by constant pressure or variable pressure.

9. The method of claim 8, wherein the cells are passed through the pores under a pressure of about 2psi, or about 2.5psi, or about 3psi, or about 5psi, or about 10psi, or about 20psi.

10. The method of any one of claims 1-9, wherein the cells pass through the pores at a uniform cell speed, or a fluctuating cell speed; wherein the cells pass through the pores at a speed ranging from about 0.1mm/s to about 20m/s.

11. The method of any one of claims 1-10, wherein the surface is contained within a larger module.

12. The method of any one of claims 1-11, wherein the viscosity of the cell suspension ranges from about 8.9×10⁻⁴ Pa.s to about 4.0×10⁻³ Pa.s and/or wherein the cell concentration ranges from about 10⁶ to about 10¹² cells/ml.

13. The method of any one of claims 1-12, wherein the method further comprises the step of passing the cell through an electric field generated by at least one electrode in proximity to the surface.

## Patentansprüche

1. Verfahren zum Abgeben einer Verbindung in eine Zelle, wobei das Verfahren das Hindurchführen einer Zellsuspension durch eine Oberfläche, die Poren enthält, umfasst, wobei die Poren die Zelle verformen, wobei dadurch eine Störung der Zelle derart bewirkt wird, dass die Verbindung in die Zelle eintritt, wobei die Zellsuspension mit der Verbindung in Kontakt gebracht wird, wobei ferner die Zellsuspension umfasst:
(i) eine gemischte Zellpopulation, wobei die Zellsuspension Vollblut, Lymphe ist oder periphere mononukleäre Blutzellen umfasst; oder
(ii) eine gereinigte primäre Zellpopulation, wobei die primäre Zelle eine Immunzelle, eine Stammzelle, eine Tumorzelle, ein Fibroblast, eine Hautzelle, ein Neuron oder eine rote Blutzelle ist;
wobei ferner die Oberfläche eine Membran und/oder ein Filter ist.

2. Verfahren nach Anspruch 1, wobei die Zellsuspension periphere mononukleäre Blutzellen umfasst.

3. Verfahren nach Anspruch 1, wobei die Zellsuspension eine gereinigte primäre Zellpopulation umfasst, wobei die primäre Zelle eine rote Blutzelle ist.

4. Verfahren nach Anspruch 1, wobei die gereinigte primäre Zellpopulation eine Immunzelle umfasst, die aus einer T-Zelle, einer B-Zelle, einer dendritischen Zelle, einem Monozyten, einem Makrophagen, einem Eosinophilen, einem Basophilen, einer NK-Zelle, einer NKT-Zelle, einer Mastzelle oder einem Neutrophilen ausgewählt ist.

5. Verfahren nach einem der Ansprüche 1-4, wobei:
(i) die Oberfläche Polycarbonat umfasst;
(ii) die Oberfläche durch Ätzen gefertigt wird;
(iii) die Querschnittsbreite der Poren im Bereich von etwa 0,4 bis etwa 35 µm liegt; oder
(iv) die Oberfläche etwa 0,1 µm bis etwa 5 mm dick ist.

6. Verfahren nach einem der Ansprüche 1-5, wobei die Oberfläche mit einem Material beschichtet ist.

7. Verfahren nach einem der Ansprüche 1-6, wobei die Verbindung:
(i) eine Nukleinsäure umfasst;
(ii) einen Polypeptid-Nukleinsäure-Komplex umfasst;
(iii) eine Nukleinsäure umfasst, die für ein Cas9-Protein und eine Leit-RNA oder Donor-DNA kodiert;
(iv) ein Protein oder Peptid umfasst
(v) ein kleines Molekül ist;
(vi) ein Nanopartikel ist;
(vii) ein chimärer Antigenrezeptor ist;
(viii) ein Liposom ist; oder
(ix) ein fluoreszenzmarkiertes Molekül ist.

8. Verfahren nach einem der Ansprüche 1-7, wobei die Zellsuspension vor, gleichzeitig oder nach dem Hindurchführen durch die Pore mit der Verbindung in Kontakt gebracht wird und/oder die Zellen unter konstantem Druck oder variablem Druck durch die Poren hindurchgeführt werden.

9. Verfahren nach Anspruch 8, wobei die Zellen unter einem Druck von etwa 2 psi oder etwa 2,5 psi oder etwa 3 psi oder etwa 5 psi oder etwa 10 psi oder etwa 20 psi durch die Poren hindurchgeführt werden.

10. Verfahren nach einem der Ansprüche 1-9, wobei die Zellen durch die Poren mit einer gleichmäßigen Zellgeschwindigkeit oder einer fluktuierenden Zellgeschwindigkeit hindurchgeführt werden, wobei die Zellen durch die Poren mit einer Geschwindigkeit im Bereich von etwa 0,1 mm/s bis etwa 20 m/s hindurchgeführt werden.

11. Verfahren nach einem der Ansprüche 1-10, wobei die Oberfläche in einem größeren Modul enthalten ist.

12. Verfahren nach einem der Ansprüche 1-11, wobei die Viskosität der Zellsuspension im Bereich von etwa 8,9 × 10⁻⁴ Pa.s bis etwa 4,0 × 10⁻³ Pa.s liegt und/oder wobei die Zellkonzentration im Bereich von etwa 10⁶ bis etwa 10¹² Zellen/ml liegt.

13. Verfahren nach einem der Ansprüche 1-12, wobei das Verfahren ferner den Schritt des Hindurchführens der Zellen durch ein elektrisches Feld umfasst, das von wenigstens einer Elektrode in der Nähe der Oberfläche erzeugt wird.

## Revendications

1. Procédé d'administration d'un composé dans une cellule, le procédé comprenant le passage d'une suspension cellulaire à travers une surface contenant des pores, lesdits pores déformant la cellule, provoquant ainsi une perturbation de la cellule de sorte que le composé entre dans la cellule, ladite suspension cellulaire étant mise en contact avec le composé ; dans lequel, en outre, la suspension cellulaire comprend :
(i) une population cellulaire mixte, ladite suspension cellulaire étant du sang total, de la lymphe ou comprenant des cellules mononucléaires de sang périphérique ; ou
(ii) une population cellulaire primaire purifiée, ladite cellule primaire étant une cellule immunitaire, une cellule souche, une cellule tumorale, un fibroblaste, une cellule cutanée, un neurone ou un érythrocyte ;
et dans lequel en outre la surface est une membrane et/ou un filtre.

2. Procédé selon la revendication 1, dans lequel la suspension cellulaire comprend des cellules mononucléaires de sang périphérique.

3. Procédé selon la revendication 1, dans lequel la suspension cellulaire comprend une population cellulaire primaire purifiée, ladite cellule primaire étant un érythrocyte.

4. Procédé selon la revendication 1, dans lequel la population cellulaire primaire purifiée comprend une cellule immunitaire sélectionnée parmi une cellule T, une cellule B, une cellule dendritique, un monocyte, un macrophage, un éosinophile, un basophile, une cellule NK, une cellule NKT, un mastocyte et un neutrophile.

5. Procédé selon l'une quelconque des revendications 1 à 4, dans lequel :
(i) la surface comprend du polycarbonate ;
(ii) la surface est fabriquée par gravure ;
(iii) la largeur de section transversale des pores varie d'environ 0,4 à environ 35 µm; ou
(iv) la surface fait d'environ 0,01 µm à environ 5 mm d'épaisseur.

6. Procédé selon l'une quelconque des revendications 1 à 5, dans lequel la surface est revêtue d'un matériau.

7. Procédé selon l'une quelconque des revendications 1 à 6, dans lequel le composé :
(i) comprend un acide nucléique ;
(ii) comprend un complexe acide nucléique-polypeptide ;
(iii) comprend un acide nucléique codant pour une protéine Cas9 et un ARN guide ou un ADN donneur ;
(iv) comprend une protéine ou un peptide
(v) est une petite molécule ;
(vi) est une nanoparticule ;
(vii) est un récepteur antigénique chimérique ;
(viii) est un liposome ; ou
(ix) est une molécule marquée par fluorescence.

8. Procédé selon l'une quelconque des revendications 1 à 7, dans lequel ladite suspension cellulaire est mise en contact avec le composé avant, pendant ou après son passage à travers le pore et/ou les cellules sont passées à travers les pores par pression constante ou pression variable.

9. Procédé selon la revendication 8, dans lequel les cellules sont passées à travers les pores sous une pression d'environ 2 psi, ou d'environ 2,5 psi, ou d'environ 3 psi, ou d'environ 5 psi, ou d'environ 10 psi ou d'environ 20 psi.

10. Procédé selon l'une quelconque des revendications 1 à 9, dans lequel les cellules passent à travers les pores à une vitesse cellulaire uniforme, ou une vitesse cellulaire fluctuante ; les cellules passant à travers les pores à une vitesse variant d'environ 0,1 mm/s à environ 20 m/s.

11. Procédé selon l'une quelconque des revendications 1 à 10, dans lequel la surface est contenue à l'intérieur d'un module de plus grandes dimensions.

12. Procédé selon l'une quelconque des revendications 1 à 11, dans lequel la viscosité de la suspension cellulaire varie d'environ 8,9 × 10⁻⁴ Pa.s à environ 4,0 × 10⁻³ Pa.s et/ou dans lequel la concentration cellulaire varie d'environ 10⁶ à environ 10¹² cellules/ml.

13. Procédé selon l'une quelconque des revendications 1 à 12, le procédé comprenant en outre l'étape consistant à faire passer la cellule à travers un champ électrique généré par au moins une électrode à proximité de la surface.
